# DEMANDE DE BREVET EUROPEEN

(11) **EP 2 653 164 A1**
(43) Date de publication de la demande: **23.10.2013**
(21) Numéro de dépôt: 12181802.5
(22) Date de dépôt: 30.03.2007
(51) Int. Cl.: A61K 31/715, A61P 31/04, C12P 19/04

(54) **Polysaccharides capsulaires de type 5 et de type 8 des souches surproductrices de S. aureus**

(30) Priorité: 31.03.2006 FR 0602795
(62) Demande divisionnaire de: 07731229.6
(71) Demandeur: Sanofi Pasteur, 69367 Lyon Cedex 07 (FR)
(72) Inventeur: Talaga, Philippe, 69280 Sainte Consorce (FR); Adam, Olivier, 69210 Lentilly (FR); Rokbi, Bachra, 69003 Lyon (FR); Meric, Claude, 69005 Lyon (FR); Mistretta, Noëlle, 69210 Sain Bel (FR)

(57) **Abrégé**

L'invention concerne les polysaccharides capsulaires de type 5 et de type 8 produits par les souches de *S. aureus* surproductrices, ainsi que les compositions immunogènes et les vaccins comprenant lesdits polysaccharides capsulaires.

## Description

L'invention concerne les polysaccharides capsulaires de type 5 et de type 8 produits par les souches de *S*. *aureus* surproductrices, en particulier par les souches CYL1892 et CYL770, ainsi que leur utilisation pour une immunisation contre une infection par une souche de *S*. *aureus* de sérotype 5 et/ou 8.

Les souches de S. *aureus* comprennent une paroi de peptidoglycane et éventuellement des polysaccharides liés à la surface externe de cette dernière qui forment une structure plus ou moins épaisse que l'on appelle capsule ou glycocalix. Ces polysaccharides capsulaires sont formés par l'assemblage d'unités répétitives dont les constituants et les liaisons sont définis et sont caractéristiques de l'espèce bactérienne. Ces unités répétitives contiennent les épitopes et les structures déterminant l'antigénicité.

La découverte de polysaccharides capsulaires sérologiquement distincts à la surface d'isolats cliniques de *S*. *aureus* a conduit à étudier leur rôle dans la virulence de ces souches. Le sérotypage des souches de *S*. *aureus* a montré que les sérotype 5 et 8 sont responsables de la grande majorité des infections humaines.

Le développement d'un vaccin contre une infection par *S*. *aureus* est un enjeu majeur du fait de la gravité des complications engendrées et de l'émergence toujours croissante de souches résistantes aux antibiotiques actuels. Le développement d'un vaccin protégeant contre les infections par des souches de *S. aureus* de sérotypes 5 et 8 (T5 et T8) permettrait de protéger un individu d'une infection par 85 à 90 % des souches de S. *aureus.*

Un obstacle important au développement d'un vaccin contre *S. aureus* basé sur les antigènes capsulaires est la faible productivité, variable en fonction des conditions de culture, des souches sauvages qui expriment les T5 et T8.

Les inventeurs ont mis en évidence de façon surprenante que les souches de S. aureus sauvages qui ont été génétiquement modifiées pour produire du polysaccharide capsulaire (PS) en grandes quantités peuvent être avantageusement utilisées pour produire des polysaccharides d'intérêt.

Les inventeurs ont en effet montré que les souches de S. aureus T5 et T8, modifiées par recombinaison génétique pour obtenir des souches surproduisant le PS capsulaire, sont capables de produire en grandes quantités dans le surnageant de culture un polysaccharide capsulaire qui, bien que différent du PS sauvage, induit une réponse humorale comprenant des anticorps contre le PS capsulaire sauvage.

La présente invention a donc pour objet une composition immunogène comprenant un polysaccharide capsulaire T8 et/ou T5 d'une souche de *S. aureus* suproductrice T8 et/ou T5 respectivement.

Selon un mode de réalisation particulier ladite composition comprend un polysaccharide capsulaire T5 d'une souche de *S. aureus* CYL1892.

Selon un autre mode de réalisation ladite composition comprend un polysaccharide capsulaire T8 d'une souche de *S. aureus* CYL770.

Selon un mode de réalisation particulier ladite composition comprend un polysaccharide capsulaire de T5 d'une souche de *S. aureus* CYL1892 et un polysaccharide capsulaire de T8 d'une souche de S. *aureus* CYL770.

Selon un mode de réalisation particulier ladite composition immunogène comprend un conjugué comprenant un polysaccharide capsulaire T8 et/ou T5 d'une souche surproductrice de S. aureus lié à une protéine porteuse.

Selon un mode de réalisation particulier ledit conjugué comprend un polysaccharide capsulaire T8 de la souche CYL770.

Selon un autre mode de réalisation ledit conjugué comprend un polysaccharide capsulaire T5 de la souche CYL1892.

Selon un mode de réalisation particulier ladite composition immunogène comprend un conjugué comprenant un polysaccharide capsulaire T8 de la souche CYL770 et un conjugué comprenant un polysaccharide capsulaire T5 de la souche CYL1892.

Selon un mode de réalisation particulier lesdits conjugués comprennent un polysaccharide capsulaire fractionné.

Selon un mode de réalisation particulier la molécule porteuse du conjugué selon la présente invention est l'exotoxine A de *Pseudomonas aeruginosa.*

Selon un autre aspect, la présente invention concerne un conjugué comprenant un polysaccharide capsulaire T8 ou T5 d'une souche surproductrice de *S. aureus* lié à une protéine porteuse, avantageusement l'exotoxine A de *Pseudomonas aeruginosa.* Selon, un mode de réalisation ledit PS est fractionné.

Selon un mode de réalisation particulier ledit conjugué comprend un polysaccharide capsulaire de la souche de *S. aureus* CYL1892.

Selon un autre mode de réalisation ledit conjugué comprend un polysaccharide capsulaire de la souche de *S. aureus* CYL770.

Lesdits PS sont de préférence fractionnés avant d'être conjugués.

Selon un autre aspect, la présente invention concerne un polysaccharide capsulaire de type 5 de la souche de S. *aureus* CYL1892. Ce polysaccharide est avantageusement sous forme isolée ou purifiée.

Selon un autre aspect, la présente invention concerne l'utilisation d'un conjugué tel que défini ci-dessus pour la fabrication d'une composition immunogène pour l'immunisation contre les infections par *S. aureus.*

Selon un autre aspect, la présente invention concerne un procédé de production de polysaccharide capsulaire T5 comprenant les étapes de :
a) culture d'une souche surproductrice de *S. aureus*
b) inactivation de la culture ainsi produite, et
c) récupération du polysaccharide capsulaire T5 à partir du surnageant.

Selon un mode de réalisation particulier du procédé selon l'invention, la souche surproductrice est la souche CYL1892.

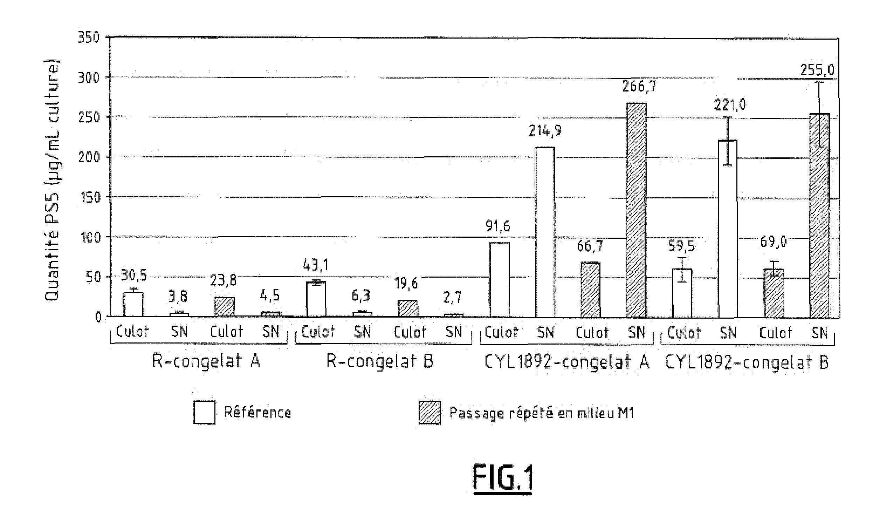

L'invention va être décrite de façon plus détaillée dans la description qui suit par référence aux figures 1 à 6.

On entend par «souche(s) de *S. aureus»* toute souche de S.aureus exprimant une capsule ou une microcapsule comprenant un polysaccharide T5 et/ou T8. On peut citer à titre d'exemple non limitatif les souches Newman (T5), Reynolds (T5), Lowenstein (T5), Becker (T8), Wright (T8). Les souches avantageusement utilisées dans le cadre de la présente invention sont les souches Reynolds et Becker.

On entend dans le cadre de la présente invention par « souche surproductrice» une souche de S. aureus qui a été modifiée génétiquement pour produire au moins 10, avantageusement au moins 20 fois plus de polysaccharide capsulaire en poids par unité de volume dans le surnageant de culture, que la souche parente dont elle est issue lorsque les deux souches sont cultivées dans les mêmes conditions de culture.

La quantité de polysaccharide capsulaire produite peut être déterminée par la méthode ELISA telle que décrite à l'exemple 1.

Une souche « génétiquement modifiée » est une souche de *S. aureus* dont le génome a été modifié par génie génétique pour augmenter sa capacité de production en polysaccharide capsulaire. A titre d'exemple illustratif d'une modification génétique appropriée, on peut citer la modification des souches de S. aureus par remplacement par recombinaison homologue du promoteur principal de l'opéron *cap5* ou *cap8* par un promoteur principal fort d'une autre souche tel que le promoteur principal de l'opéron *cap1* de la souche M de *S. aureus.* Le procédé d'insertion de gène par recombinaison homologue est décrit en détails dans l'article de A. J. Link et al (J. of Bacteriology. Oct. 1997, p.6228-6237) et son application spécifique à la production d'une souche de S. aureus surproductrice est décrite en détails dans l'article de T.T. Luong et al (Infection and Immunity, Juillet 2002, p3389-3395). On peut donc se référer à ces articles pour plus de détails sur les conditions opératoires utilisables.

Ce même procédé peut également être utilisé pour construire une souche recombinante surproduisant le polysaccharide capsulaire T5. Il peut être pour ce faire nécessaire d'adapter certains paramètres, la recombinaison homologue étant plus ou moins facile à mettre en oeuvre en fonction de la souche de départ. A titre d'exemple, une souche Reynolds CYL1892 surproductrice dans laquelle le promoteur principal de l'opéron cap5 a été remplacé par recombinaison homologue par le promoteur principal de l'opéron cap1 de la souche M de S. aureus a pu être produite par le procédé de Luong et al dans lequel les modifications suivantes ont été apportées : les séquences homologues ont été remplacées par des séquences équivalentes homologues de la souche Reynolds, le plasmide pCL52.2 a été remplacé par le plasmide pCL10 contenant une origine de réplication thermosensible et comprenant un marqueur cat (chloramphenicol acetyl tranferase conférant une résistance au chloramphénicol). Brièvement un fragment d'ADN d'environ 1 kb contenant l'ORF *cap5* de la souche Reynolds fusionné à un fragment d'environ 250-bp contenant le P*cap1* promoteur est construit par la technique de PCR par chevauchement. Un fragment d'ADN d'environ 1 kb contenant la séquence amont du gène *cap5A* est amplifié par PCR à partir du génome de la souche Reynolds. Les deux fragments sont ensuite clonés dans le plasmide pCL10 portant une origine de réplication thermosensible et comportant un marqueur cat de sorte que le promoteur P*cap5* soit remplacé par le promoteur P*cap1.* Le plasmide ainsi construit est ensuite électroporé dans une souche RN4220 puis introduit par transduction à l'aide du bactériophage 52 dans la souche Reynolds. La sensibilité à la température de l'origine de réplication du plasmide permet ensuite de favoriser la recombinaison homologue en bloquant sa réplication tout en appliquant une sélection par le chloramphénicol.

Par «souche CYL770», on désigne la souche de *S. aureus* surproductrice, obtenue à partir d'une souche sauvage Becker (CIP103314) dans laquelle le promoteur de l'opéron *cap8* a été remplacé par le promoteur fort de l'opéron cap1 de la souche M *de S. aureus,* comme décrit par Luong et Lee (2002 Infect.Immun. 70: 3389-3395).

Par « souche CYL1892 », on désigne la souche de *S. aureus* surproductrice, obtenue à partir d'une souche Reynolds (CIP 103313) dans laquelle le promoteur de l'opéron *cap5* a été remplacé par le promoteur constitutif fort de l'opéron *cap1* de la souche M de *S. aureus,* selon le procédé décrit par Luong et Lee (2002 Infect.Immun. 70: 3389-3395) comprenant les modifications décrites ci-dessus.

Les inventeurs ont également mis en évidence que les souches surproductrices de S. aureus, en particulier les souches CYL770 et CYL1892 présentent une stabilité génétique élevée. On entend par stabilité génétique élevée la propriété des souches à conserver leur capacité de surproduction après au moins 25 passages sur un milieu de culture approprié à la culture des souches de S. aureus capsulées, telle que mesurée par le test décrit dans l'exemple 1.

D'autre part les inventeurs ont montré que le polysaccharide capsulaire produit par les souches surproductrices de S. aureus (appelé par la suite rPS) présente des caractéristiques physico-chimiques différentes de celles du PS produit par la souche sauvage correspondante (appelé par la suite sPS). Les termes « PS8 » et « PS T8 » sont utilisés indifféremment dans le cadre de la présente demande pour désigner un polysaccharide capsulaire de sérotype 8. De même, les termes « PS5 » et « PS T5 » sont utilisés indifféremment dans le cadre de la présente demande pour désigner un polysaccharide capsulaire de sérotype 5.

Le polysaccharide rPS T8 présente un poids moléculaire moyen très supérieur au sPS correspondant i.e. 250 kDa versus 150 kDa.

Le polysaccharide rPS T5 présente un poids moléculaire moyen très supérieur au sPS correspondant i.e. 150 kDa versus 50 kDa. D'autre part, contrairement au sPS T5 qui reste lié à la paroi bactérienne, les inventeurs ont montré que le rPS T5 est majoritairement relargué dans le surnageant de culture.

Les inventeurs ont également observé que les rPS présentent des propriétés d'adsoption sur une surface plastique différentes de celles des sPS. Des anticorps polyclonaux anti-PS8 ont été générés chez le lapin par injections répétées de différentes souches T8 (Becker, Wright et CYL770), préalablement cultivées en milieu M1. Les titres en anticorps anti-PS8 de ces sera ont été évalués par ELISA en utilisant différents PS8 (purifiés à partir des souches Becker, Wright ou CYL770) comme antigène d'adsorption. Les résultats ont montré que le sérum polyclonal anti-PS8, généré à partir de la souche Becker, présente un fort titre en anticorps anti-PS8. Ce titre est similaire, qu'il soit évalué sur le PS8 de la souche Becker ou sur le PS8 de la souche Wright. Par contre, un faible titre anti-PS8 est observé lorsque celui-ci est évalué avec du PS8 adsorbé qui a été purifié à partir de la souche CYL770. Les mêmes résultats ont été obtenus pour les sera générés à partir des souches Wright ou CYL770.

Pour vérifier que la faible reconnaissance du PS8 de CYL770 par les différents sera était due à une capacité moindre de ce rPS8 à se fixer sur le plastique traité d'une plaque ELISA, et non à une différence de reconnaissance du PS8 lui-même, les trois sera ont été titrés en ELISA sur du rPS8 de CYL770 conjugué à la rEPA, la présence de la protéine porteuse devant améliorer grandement l'adsorption. Dans ces conditions d'adsorption, les sera polyclonaux anti-PS8 générés à partir des 3 souches T8 différentes, reconnaissent très fortement le rPS8 de CYL770, avec des titres en anticorps comparables entre eux et similaires à ceux obtenus sur PS8 purifié à partir des souches Becker ou Wright.

Malgré ces différences importantes, pouvant potentiellement se traduire par des propriétés immunogéniques et antigéniques différentes, les inventeurs ont mis en évidence par des expériences de caractérisation et d'immunogénicité que les rPS conservaient d'une part leur structure primaire caractéristique telle que décrite par C. Jones (Carbohydr. Res., 2005, 340, 1097-1106) et conservaient d'autre part l'immunogénicité et l'antigénicité des sPS.

Les Inventeurs ont en particulier évalué la réactivité croisée d'un sérum anti-CYL770 contre différentes souches de *S. aureus* de type 8. Pour cela, un sérum hyperimmun a été préparé chez le lapin par injection de germes entiers formolés de la souche CYL770 et adsorbé sur germes autologues chimiquement acapsulés. Les résultats obtenus montrent que le sérum anti-souche CYL770 présente une réactivité croisée avec les souches sauvages Becker et Wright présentant à leur surface un sPS T8. Ces résultats démontrent donc qu'il est possible d'utiliser la souche surproductrice CYL770 pour produire de grandes quantités d'un polysaccharide capsulaire T8 utilisable dans une méthode d'immunisation.

Les rPS peuvent être produits par un procédé de production comprenant les étapes de :
a) culture d'une souche de *S. aureus* surproductrice, en particulier une souche CYL1892 ou CYL770 ,
b) inactivation de la culture ainsi produite, et
c) récupération du polysaccharide capsulaire T5 ou T8 à partir du surnageant de culture.

L'étape (a) peut être mise en oeuvre sur un milieu de culture solide ou liquide. Tout milieu de culture décrit dans la littérature comme étant approprié à la culture d'une souche de S aureus capsulée peut être utilisé pour ce faire. On peut citer à titre d'exemple non limitatif les milieux suivant : Bouillon TSB (trypticase soja) ; milieu défini de Poutrel (Poutrel et al., Clin Diagn Lab Immunol. 1995 2(2):166-71) ; Bouillon Columbia. Il est fait référence par la suite à des milieux inducteurs ou non inducteurs. On entend par « milieu inducteur » un milieu de culture qui contient un élément (par exemple NaCl ou un autre sel tel que CaCl₂ et MgCl₂) qui agit indirectement ou directement sur le promoteur principal inductible du locus cap. Un milieu inducteur est donc un milieu capable d'induire la production de capsule chez les souches de S. aureus comprenant un promoteur principal inductible dans le locus cap. Par opposition un milieu non inducteur est un milieu de culture qui ne contient pas un tel signal.

On utilisera de préférence dans le cadre de la présente invention un milieu dépourvu de protéine d'origine animale.

Par « protéine d'origine animale » on entend les protéines obtenues à partir de matière d'origine animale ainsi que touts produits en dérivant, tels que les dérivés issus du traitement chimique des protéines animales. On entend également les produits de l'hydrolyse partielle ou totale de ces protéines animales tels que les peptones, les polypeptides, les peptides, ou les acides aminés en dérivant.

L'étape de culture peut être réalisée par ensemencement du milieu de culture avec un inoculum, par exemple avec une DO₆₈₀ₙₘ initiale de 0,2, et incubation à 37°C pendant une durée par exemple d'environ 48 à 72 heures dans une atmosphère contenant ou pas du CO₂ à une concentration de 5 à 10% (pour une souche T5 sans CO₂ et dans le cas de l'utilisation d'une souche T8 avec CO₂). Le volume de culture peut varier en fonction des besoins. Des volumes de 400ml à 201 ou des volumes supérieurs par exemple de 301 à 1001 peuvent être utilisés. De préférence un rapport volume du milieu de culture par rapport au volume du récipient de culture de 20 % (V/V) est maintenu pour favoriser l'oxygénation. Pour les grands volumes, l'oxygénation est contrôlée et régulée au cours de la culture par un ajustement de la pression en oxygène et une agitation appropriée.

En fonction des besoins, l'étape de culture peut comprendre des cultures successives dans des volumes croissants afin d'augmenter la biomasse et la quantité de polysaccharide qui sont produites. La culture résultant de l'étape (a) est ensuite soumise à une étape d'inactivation (b).

L'étape d'inactivation peut être mise en oeuvre par traitement de la culture avec un mélange phénol/éthanol (1V/1V) à raison de 2 % en volume au final, puis agitation par exemple à l'aide d'un barreau aimanté à température ambiante pendant 6 heures à 48 heures en fonction de la biomasse à traiter. L'inactivation est contrôlée par un test de mortalité. Pour ce faire, 100 microlitres de la culture traitée sont étalés sur une gélose Columbia 2 % NaCl et en parallèle 100 microlitres de la culture traitée sont ensemencés en bouillon trypcase soja additionné de 1/30^{ème} de sérum de cheval. Les cultures sont incubées pendant 48 heures à 37°C, les bouchons des flacons de bouillon restant dévissés. L'absence de bactéries viables se traduit par une absence de pousse après 24 heures d'incubation à 37°C. Une fois que la mortalité de la culture est établie, le polysaccharide capsulaire est récupéré.

Dans le cas des souches surproductrices de S. aureus, en particulier CYL770 et CYL1892, la plus grande partie des rPS T8 et T5, est relarguée dans le surnageant de culture. Les rPS peuvent donc être isolés directement à partir de ce dernier. Pour ce faire, à l'issue de l'étape d'inactivation, le milieu est centrifugé et le culot cellulaire est éliminé.

Le rPS contenu dans le surnageant de culture peut ensuite être purifié par toute technique classique de purification de PS. Différentes techniques de purification ont été décrites dans la littérature et peuvent être utilisées dans le cadre de la présente invention. On peut se référer par exemple aux articles de Lee J.C. et al 1987 ; 55 :2191-2197 ou de Fattom et al Infect. Immun. 1990 ; 58 :2367-2374, ce dernier décrivant un procédé impliquant une libération des PSs par la lysostaphine, traitements par RNAse, DNAse, protéase, précipitation éthanolique fractionnée, échange d'ions et tamisage moléculaire. On utilisera de préférence dans le cadre de la présente invention une technique conduisant à un rPS présentant un taux de pureté d'au moins 70%.

Les analyses physico-chimiques et l'analyse par résonance magnétique nucléaire du proton à 500 MHz à une dimension montrent que les rPS T8 et T5 purifiés à partir d'une souche surproductrice présentent une structure conforme à celle décrite dans la littérature avec un taux de pureté d'au moins 70 % et un taux de O- et N- acétylation supérieur à 70 %. Les résiduels sont présents dans le produit final à raison de moins de 5 % (p/p) de résiduels de nature protéique, moins de 1% (p/p) d'acides nucléiques résiduels et moins de 5% (p/p) d'acide lipoteichoïque et d'acide teichoïque résiduels.

La quantité de polysaccharide capsulaire obtenue peut être évaluée par la technique ELISA telle que décrite dans l'exemple 1.

Il a ainsi été montré que la souche CYL770 produisait dans le surnageant une quantité de rPS T8 environ 80 fois supérieure à celle produite par la souche parente Becker. Ce gain de productivité reste stable après des passages successifs en milieu de culture

Les Inventeurs ont par ailleurs montré que la souche CYL1892 produisait environ 50 fois plus de rPS T5, soit 200 µg/ml en moyenne, que la souche sauvage parente. Ce gain de productivité a été essentiellement observé dans le surnageant de culture et reste stable après des passages successifs en milieu de culture. Par ailleurs, la souche CYL1892 produit 7 fois plus de polysaccharide T5 au niveau du culot cellulaire que la souche parente sauvage.

Une partie du rPS T5 restant liée à la paroi bactérienne, l'étape de récupération du rPS T5 peut également comprendre une étape supplémentaire d'extraction du rPS T5 à partir des cellules. Différentes techniques d'extraction ont été décrites dans la littérature pour ce faire et peuvent être utilisées dans le cadre de la présente invention. On peut se référer par exemple aux articles de Lee J.C. Infect. Immun. 1993 ; 67:1853-1858 ; Dassy B. et al. Gen. Microbiol. 1991 ; 137:1155-1162 pour un descriptif détaillé de ces techniques.

Le procédé de production de PS selon l'invention peut donc être utilisé avantageusement pour produire du rPS T5, de préférence à partir d'une souche CYL1892, directement à partir du surnageant d'extraction du PS à partir du culot cellulaire. Le procédé selon l'invention permet donc de simplifier les étapes lourdes d'élimination des nombreux contaminants cellulaires que sont par exemple : DNA, RNA, acide teichoïque.

Selon un mode de réalisation, l'invention a aussi pour objet un polysaccharide capsulaire rPS, en particulier un rPS T5 de la souche de *S. aureus* CYL1892. Ledit polysaccharide est avantageusement sous forme isolé ou purifié.

Les rPS T5 et T8 selon l'invention peuvent être fractionnés ou dépolymérisés par exemple selon le procédé décrit dans le brevet US 6,045,805, auquel on peut se référer pour un descriptif complet des conditions opératoires. Dans le cadre de la présente invention, on utilise indifféremment les termes «fractionner» et «dépolymériser». Ces rPS fractionnés sont nommés ci-après rPSf. Les rPS T5 et T8 selon l'invention sont avantageusement fractionnés ou dépolymérisés jusqu'à l'obtention d'un rPSf présentant un poids moléculaire moyen avantageusement compris dans une gamme allant de 10 à 120 kDa, en particulier dans une gamme allant de 30 à 70 kDa tel qu'un rPSf de PM moyen de 50kDa.

Selon un mode de réalisation, l'invention a donc également pour objet un polysaccharide capsulaire rPSf , en particulier un rPSf T5, avantageusement un rPSf T5 de la souche de *S. aureus* CYL1892. Ledit polysaccharide rPSf est avantageusement sous forme isolé ou purifié.

L'invention concerne également l'utilisation des polysaccharides capsulaires rPS T5 et T8 produits par les souches de S. *aureus* surproductrices, avantageusement les souches CYL1892 et CYL770, pour produire des compositions immunogènes et des vaccins pour le traitement ou la prévention des infections par les souches de S. *aureus* T5 et/ou T8.

Les polysaccharides capsulaires rPS induisent la production d'anticorps pouvant médier la destruction des bactéries qui les portent, par des phagocytes en présence de complément.

Selon un mode de réalisation, l'invention concerne donc une composition immunogène ou vaccinale comprenant un rPS T8 et/ou T5 d'une souche de S. aureus surproductrice, avantageusement d'une souche CYL770 ou CYL1892.

L'invention concerne également l'utilisation d'un rPS T8 et/ou T5 d'une souche de S. aureus surproductrice, avantageusement d'une souche CYL770 ou CYL1892 pour la fabrication d'une composition immunogène ou pour la fabrication d'une composition vaccinale destinée à la prévention et/ou au traitement d'une infection par *S. aureus.* En particulier, ladite composition immunogène ou vaccinale permet d'induire une réponse humorale contre les souches de *S. aureus* T8 et/ou T5 chez le sujet auquel elle est administrée.

Selon un mode de réalisation particulier, ladite composition comprend les rPS T8 et T5. Une telle composition immunogène ou vaccinale est particulièrement avantageuse puisqu'elle permet d'induire, chez le sujet auquel elle est administrée, une réponse immunitaire contre les souches *S. aureus* T5 et T8 qui représentent la très grande majorité des souches *S. aureus* infectieuses.

La composition immunogène ou vaccinale selon l'invention peut être utilisée pour combattre les infections chez des sujets infectés ou la prévenir chez des sujets à risques tels que les personnes hospitalisées ou devant subir une intervention chirurgicale.

Lorsque la composition immunogène ou vaccinale selon l'invention est destinée à un enfant de moins de 2 ans ou à un sujet présentant une défense immunitaire affaiblie, telle qu'une personne âgée (>60 ans). Le rPS selon l'invention est de préférence utilisé sous une forme conjuguée.

Selon un autre mode de réalisation, la présente invention concerne donc un conjugué rPS-protéine porteuse.

Par « protéine porteuse » on entend une protéine qui permet la collaboration cellulaire entre cellules T et B dans l'induction de la réponse immune contre le polysaccharide capsulaire et améliore ainsi l'immunogénicité que ce soit pour une immunisation active ou pour une immunisation passive utilisant des antisérums de titre élevé préparés sur des volontaires. Les protéines porteuses sont préférentiellement des protéines non toxiques et non réactogènes. Des exemples non limitatifs de protéines porteuses susceptibles d'être utilisées incluent des toxines bactériennes recombinantes ou inactivées telles que l'exotoxine A de *Pseudomonas aeruginosa,* les anatoxines tétanique, diphtérique ou coquelucheuse, des anatoxines ou exotoxines de Staphylocoques, ou encore la toxine thermolabile (LT) ou les Shiga-toxines (ST) de *E. coli.* Des protéines de la membrane externe de bactéries peuvent également être utilisées, comme par exemple le complexe c de la membrane externe (OMPc), des porines, protéines de liaison de transferrine, la protéine A de surface des pneumocoques (PsaA). D'autres protéines encore telles que l'albumine de sérum bovin (BSA), l'hémocyanine de patelle ou la tuberculine dérivée de protéine purifiée (PPD) peuvent être employées en tant que protéine porteuse.

Du fait de leur haut poids moléculaire, les polysaccharides rPS T5 et T8 sont de préférence « fractionnés » ou « dépolymérisés » avant conjugaison à la molécule porteuse, par exemple selon le procédé décrit dans le brevet US 6,045,805 auquel on peut se reporter pour un descriptif des conditions opératoires à utiliser. Les rPS non fractionnés peuvent conduire à un rendement cumulé de conjugaison et de purification plus faible et à des conjugués dont la filtration stérilisante peut être plus difficile à mettre en oeuvre du fait de leur taille plus importante. Les rPS dépolymérisés ou fractionnés sont nommés dans la suite de la demande rPSf.

Les rPS selon l'invention peuvent être dépolymérisés ou fractionnés jusqu'à l'obtention du rPSf présentant un poids moléculaire moyen de 10 à 120 KDa, en particulier de 30 à 70 KDa, tel que 50 kDa. A titre illustratif le rPS T5 de la souche CYL1892 est dépolymérisé ou fractionné jusqu'à l'obtention d'un rPSf possédant un poids moléculaire moyen avantageusement compris dans une gamme allant de 30 à 70 kDa, par exemple 50kDa et le rPS T8 de la souche CYL770 est dépolymérisé ou fractionné jusqu'à l'obtention d'un rPSf possédant un poids moléculaire moyen avantageusement compris dans une gamme allant de 30 à 70 kDa, par exemple 50kDa.

Le polysaccharide est ensuite fonctionnalisé avant d'être conjugué à la protéine porteuse par liaison covalente. La fonctionnalisation peut être effectuée selon différentes méthodes. Par exemple, des groupes carboxylates activés du polysaccharide peuvent être fonctionnalisés avec le dihydrazide d'acide adipique (ADH) ou la cystamine, puis le polysaccharide peut être conjugué à la protéine porteuse par une réaction médiée par un carbodiimide du polysaccharide partiellement amidé avec un groupe carboxylate de la protéine porteuse. Des groupes hydroxyle du polysaccharide peuvent aussi être activés à l'aide de bromure de cyanogène ou 1-cyano-4-diméthylamino-pyridium tétrafluoroborate, puis le polysaccharide peut être fonctionnalisé avec l'ADH conformément au procédé décrit par Kohn et al. (1993 FEBS Lett. 154: 209:210). Le polysaccharide fonctionnalisé est ensuite lié à la protéine porteuse, par exemple à l'exoprotéine A recombinante de *Pseudomonas aeruginosa* dans laquelle le résidu GLU 553 a été délété, à laquelle il est fait référence dans la présente demande sous le terme de rEPA, en présence d'éthyldiméthylaminopropyl-carbodiimide (EDAC). Une chromatographie d'exclusion de taille peut ensuite être utilisée pour séparer les conjugués des polysaccharides n'ayant pas réagi. Les étapes d'activation de fonctionnalisation et de conjugaison n'altèrent pas la structure chimique des unités répétitives du polysaccharide.

Selon un aspect, l'invention concerne donc un conjugué comprenant du PSf T8 et/ou T5, avantageusement de la souche CYL770 et CYL1892 respectivement, conjugué à une protéine porteuse, de préférence l'ExoA détoxifiée rEPA.

Selon un autre aspect, l'invention concerne aussi un conjugué comprenant du rPSf T8 et/ou T5, avantageusement de la souche CYL770 et CYL1892 respectivement, conjugué à une protéine porteuse, de préférence l'ExoA détoxifiée rEPA.

Selon un mode de réalisation particulier l'invention concerne un mélange comprenant les conjugués tels que définis ci-dessus, en particulier, les conjugués PSf T8 et T5 ou les conjugués rPST8 et T5, dans lequel les PS sont conjugués à une protéine porteuse identique ou différente, avantageusement identique et correspondant à l'ExoA détoxifiée.

Les conjugués selon l'invention peuvent être utilisés pour la fabrication d'une composition immunogène ou vaccinale, en particulier une composition vaccinale destinée à la prévention et/ou au traitement d'une infection par *S. aureus.* L'invention porte donc également sur une composition immunogène ou vaccinale comprenant lesdits conjugués ou leur mélange tels que définis ci-dessus.

Les compositions selon l'invention sont utiles pour induire une réponse humorale *in vivo,* notamment pour produire des anticorps ou des anti-sérums contre une souche *S. aureus* T5 ou T8, ou pour combattre l'infection chez des sujets infectés ou la prévenir chez des sujets à risque tels que des personnes hospitalisées ou devant subir une intervention chirurgicale.

La présente invention a donc également pour objet la préparation desdits anticorps et antisérums qui peut être mise en oeuvre par les techniques classiques d'obtention d'antisérums et d'anticorps polyclonaux et monoclonaux bien connues de l'homme de l'art ainsi que les anticorps et antisérums dirigés ainsi produits.

Les compositions immunogènes ou vaccinales selon l'invention peuvent être préparées par toute méthode usuelle connue de l'homme du métier. Habituellement, les antigènes selon l'invention sont mélangés avec un excipient pharmaceutiquement acceptable. Par « excipient pharmaceutiquement acceptable » on entend tout excipient, charge, diluant, véhicule, conservateur etc, qui est classiquement utilisé dans la préparation des compositions administrables à l'homme. En général, ces produits sont sélectionnés en fonction de la forme galénique choisie et de la voie d'administration et selon les pratiques pharmaceutiques standards. On peut se référer par exemple au Remington's Pharmaceutical Sciences, qui constitue un manuel de référence dans le domaine.

Les compositions selon l'invention peuvent en outre contenir un adjuvant. Tout adjuvant ou mélange d'adjuvants pharmaceutiquement acceptable classiquement utilisé dans le domaine des vaccins peut être utilisé à cette fin. On peut citer à titre d'exemple d'adjuvant approprié les sels d'aluminium tels que l'hydroxyde d'aluminium ou le phosphate d'aluminium, le DC-Chol et les agonistes de Toll.

Selon un autre aspect encore, l'invention a trait à une méthode d'immunisation d'un sujet contre une infection par S. *aureus* comprenant l'administration audit sujet d'une quantité immunoefficace d'une composition immunogène ou vaccinale selon l'invention telle que définie dans la description ci-dessus.

Par « sujet » on entend tout sujet à risque vis-à-vis d'une infection par *S. aureus,* tel que par exemple une personne hospitalisée ou devant subir une intervention chirurgicale.

Les compositions selon l'invention peuvent être administrées par toute voie classique, habituellement utilisée dans le domaine des vaccins, telle que la voie parentérale (intraveineuse, intramusculaire, sous-cutanée, etc..). On utilisera de préférence dans le cadre de la présente invention pour les compositions injectables et une administration intramusculaire. Une telle administration peut être réalisée avantageusement au niveau des muscles de la cuisse ou du bras. Les compositions selon la présente invention peuvent également être administrées par voie orale. Une administration via la muqueuse nasale, vaginale ou rectale peut être également recommandée dans le cadre de la présente invention. L'administration peut être réalisée par l'administration d'une dose unique ou de doses répétées. Une dose vaccinale peut être préparée sous un volume de 0,1 ml à 2 ml, de façon préférée sous un volume de 0,5 ml. Une quantité immunoefficace est une quantité capable d'induire, chez un sujet vacciné, une réponse humorale comprenant des anticorps capables d'opsoniser une souche de *S. aureus* et de faciliter son élimination par les phagocytes de l'individu infecté. La composition peut être avantageusement administrée au moins 10 à 14 jours avant le début de la période à risque accru d'infection par *S. aureus*

Les figures et exemples ci-dessous illustrent l'invention sans en limiter sa portée.

### FIGURES

La Figure 1 représente la quantité de polysaccharide capsulaire de type 5 (PS5) mesurée dans le culot ou le surnageant de culture pour la souche Reynolds ou la souche recombinante CYL1892.

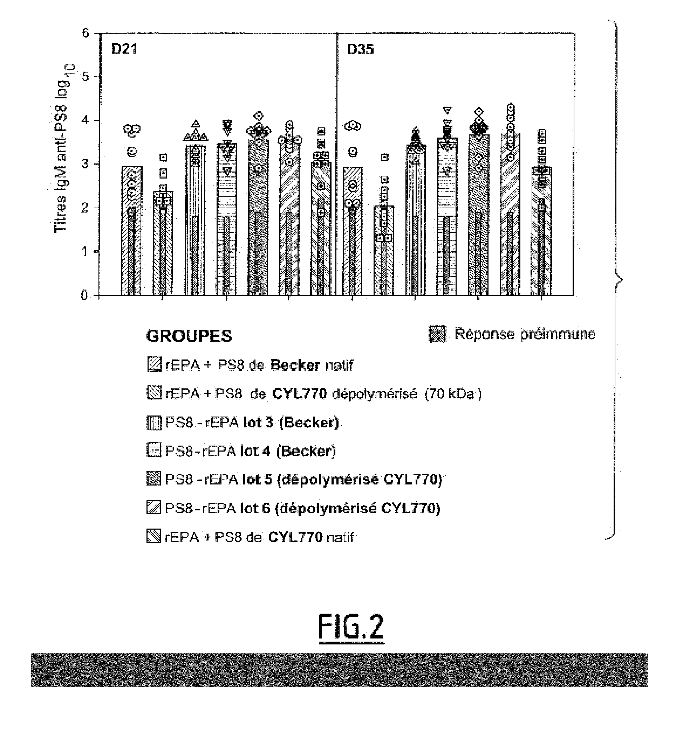

La Figure 2 représente les titres en IgM anti-PS8 induits chez des souris immunisées avec les conjugués PS T8-rEPA préparés à partir de la souche CYL770 ou de la souche Becker. La valeur des titres (histogramme) est représentée par le Log de la moyenne des inverses de dilution. Pour chacun des groupes, le titre individuel de chaque sérum (n=10) est représenté par un symbole. La réponse non spécifique mesurée chez les animaux préimmuns (J-1) est également représentée.

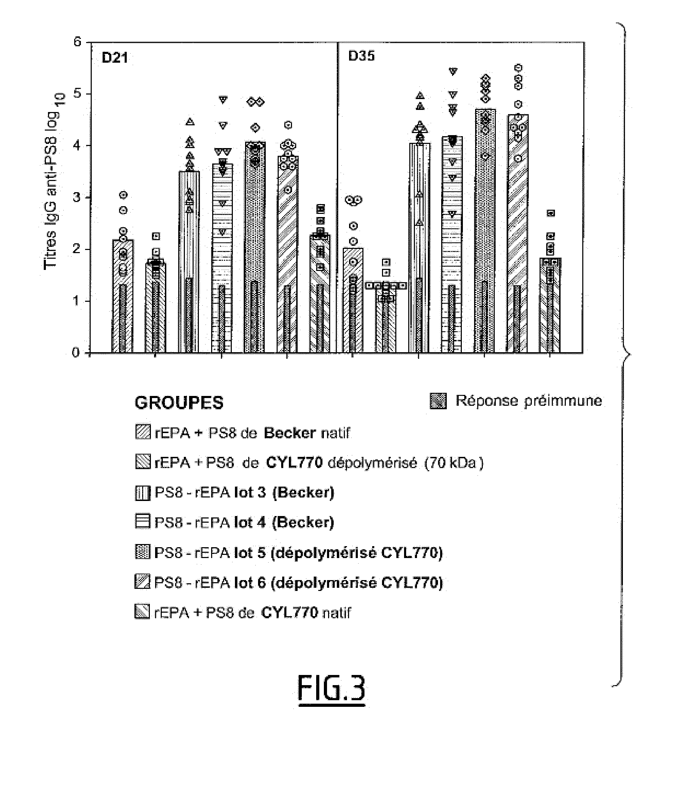

La Figure 3 représente les titres en IgG totales anti-PS8 induits chez des souris immunisées avec les conjugués PS T8-rEPA préparés à partir de la souche CYL770 ou de la souche Becker. La valeur des titres (histogramme) est représentée par le Log de la moyenne des inverses de dilution. Pour chacun des groupes, le titre individuel de chaque sérum (n=10) est représenté par un symbole. La réponse non spécifique mesurée chez les animaux préimmuns (J-1) est également représentée.

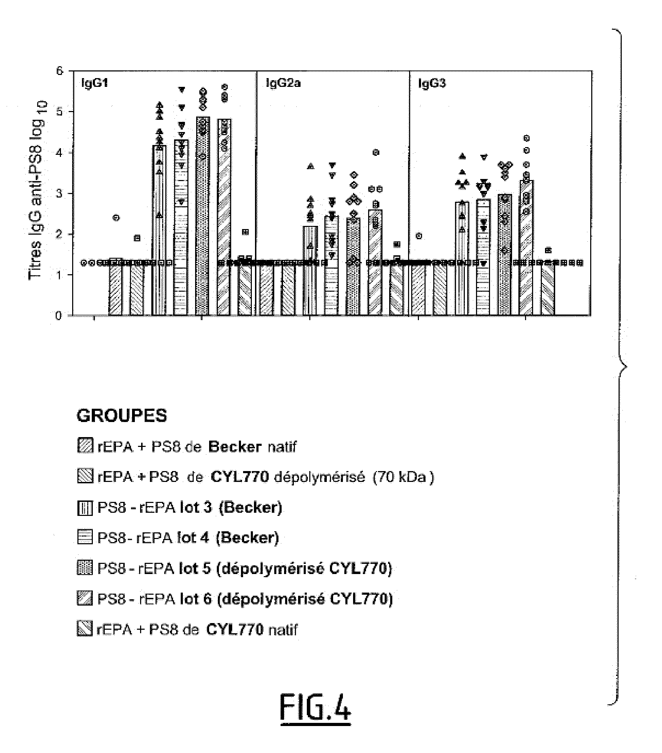

La Figure 4 représente les titres des sous-classes IgG1, IgG2a et IgG3 anti-PS8, mesurés à J35 chez des souris immunisées avec les conjugués PS T8-rEPA préparé à partir de la souche CYL770 ou de la souche Becker. La valeur des titres (histogramme) est représentée par le Log de la moyenne des inverses de dilution. Pour chacun des groupes, le titre individuel de chaque sérum (n=10) est représenté par un symbole. La réponse non spécifique mesurée chez les animaux préimmuns (J-1) est également représentée.

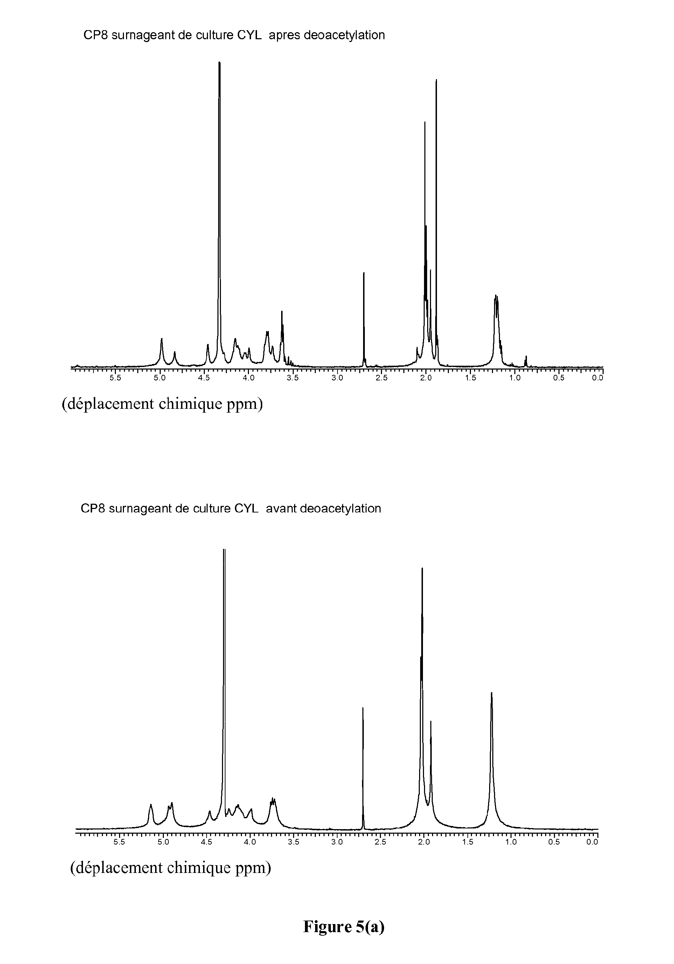

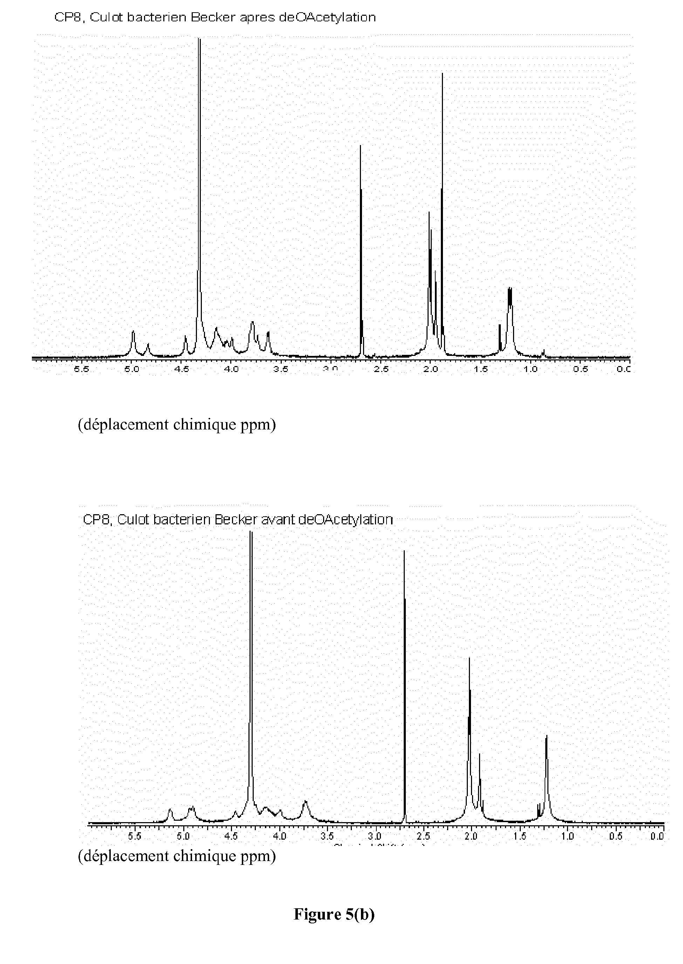

Les Figures 5(a) et (b) représentent respectivement les spectres obtenus par analyse par RMN du proton à une dimension et à 500 MHz du polysaccharide rPS8 purifié à partir d'un surnageant de la souche CYL770 et du sPS8 purifié à partir du culot cellulaire d'une culture de la souche Becker. Ces analyses par RMN ont été réalisées en utilisant un spectromètre Bruker DRX 500 et une sonde: BB-1 H-D XYZ GRD 5mm. Les échantillons ont été repris en D₂O à une concentration de 1.2mg/ml. L'analyse a été mise en oeuvre dans les conditions suivantes : température d'analyse : 343°K (70°C) ; temps de relaxation: 2s ; nombre de balayage = 512. Pour chaque PS8, les spectres ont été réalisés sur la forme native (O-acétylée) et sur la forme dé-O-acétylée obtenue par traitement alcalin tel que décrit par C. Jones (C. Jones. Carbohydr. Res. 2005, 340, 1097-1106).

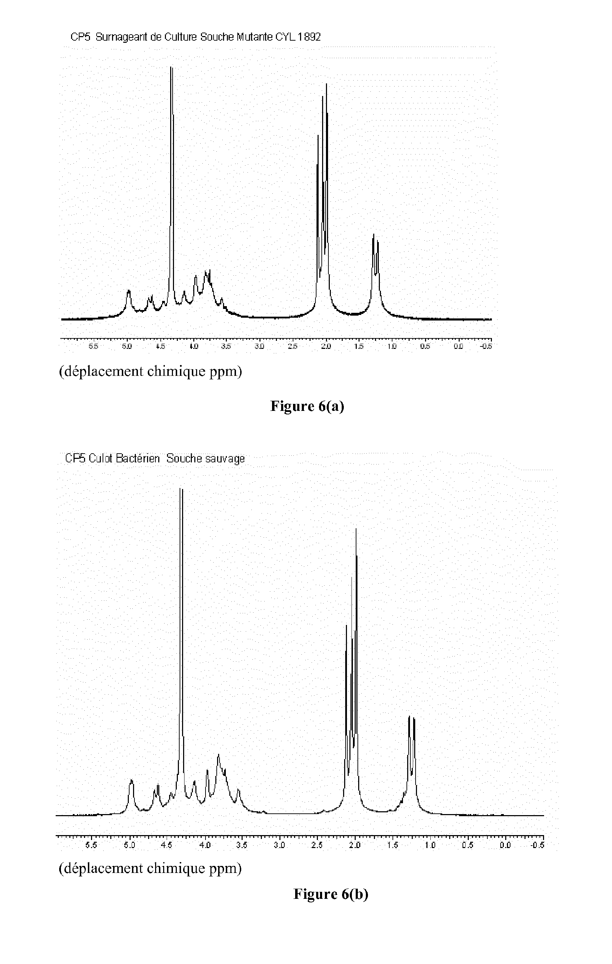

Les Figures 6(a) et (b) représentent respectivement les spectres obtenus par analyse par RMN du proton à une dimension et à 500 MHz du polysaccharide rPS5 purifié à partir du surnageant de culture de la souche CYL1892 et du sPS5 purifié à partir du culot cellulaire d'une culture de la souche Reynolds. Ces analyses par RMN ont été réalisées en utilisant un spectromètre Bruker DRX 500 et une sonde: BB-1 H-D XYZ GRD 5mm. Les échantillons ont été repris en D₂O à une concentration de 1.2mg/ml. L'analyse a été mise en oeuvre dans les conditions suivantes : température d'analyse : 343°K (70°C) ; temps de relaxation: 2s ; nombre de balayage = 512.

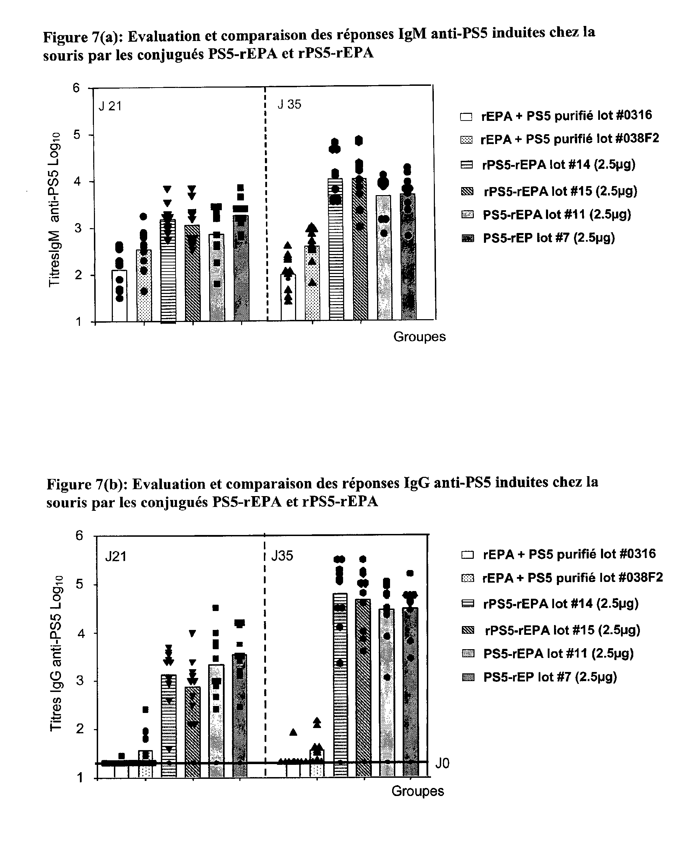

Les figures 7(a) et 7(b) représentent respectivement l'évaluation et la comparaison des réponses IgM et IgG anti-PS5 induites chez la souris par les conjugués PS5-rEPA et rPS5-rEPA.

### EXEMPLES

La composition des milieux de culture utilisés dans les exemples suivants est telle que définie dans le tableau 1 ci-dessous. Lesdits milieux ont été préparés par mélange des constituants dans l'ordre indiqué dans le tableau.

**Tableau 1 : Préparation des milieux de culture liquides et solides**

| | | Milieu M0 gélosé inducteur | | Milieu M0 gélosé non inducteur | | Milieu M0 liquide inducteur | | Milieu M1 inducteur | |
|---|---|---|---|---|---|---|---|---|---|
| Composants | Concentration Sol. mère g/l | Conc. finale g/l | Volume pour 37,5 ml en ml | Conc. finale g/l | Volume pour 37,5 ml en ml | Conc. finale g/l | Volume pour 100 ml en ml | Conc. finale g/l | Volume pour 100 ml en ml |
| Peptone blé | 387 | 3,87 | 1,5 | 3,87 | 1,5 | 30 | 7,75 | 30 | 7,75 |
| Extrait de Levure | 250 | 5 | 3 | 5 | 3 | 5 | 2 | 5 | 2 |
| D-glucose H₂O | 100 | 1 | 1,5 | 1 | 1,5 | 1 | 1 | 1 | 1 |
| NaCl | 300 | 6 | 3 | 6 | 3 | 6 | 2 | 58,5 | 19,5 |
| MgCl₂ 6H₂O | 40 | 0,4 | 1,5 | 0,4 | 1,5 | 0,4 | 1 | 0,4 | 1 |
| (NH₄)₂SO₄ | 340 | 6,8 | 3 | | | 6,8 | 2 | 6,8 | 2 |
| NH₄Cl | 300 | 6 | 3 | | | 6 | 2 | 6 | 2 |
| FeCl₃ 6H₂O | 12 | 120 x10⁻³ | 1,5 | 120 x10⁻³ | 1,5 | 120 x10⁻³ | 1 | 120 x10⁻³ | 1 |
| ZnCl₂ | 5 | 5 x10⁻³ | 0,15 | | | 5 x10⁻³ | 0,1 | 5 x10⁻³ | 0,1 |
| CaCl₂ 2H₂O | 10 | 10 x10⁻³ | 0,15 | | | 10 x10⁻³ | 0,1 | 10 x10⁻³ | 0,1 |
| Hepes 500 ml | 500 | | | 50 | 15 | | | | |
| K₂HPO₄ | 500 | | | 10 | 3 | | | | |
| KCI | 54 | | | 0,54 | 1,5 | | | | |
| MnSO₄ H₂O | 3,78 | | | 3,78 x10⁻³ | 0,15 | | | | |
| H₂O ultra filtrée | | | 19,2 | | 5,85 | | 81,05 | | 63,55 |
| TOTAL | | | 37,5 | | 37,5 | | 100 | | 100 |

### Exemple 1 : Caractérisation de la souche CYL1892 de type 5

Un congelat de la souche CYL1892 a été fourni par le Dr. Chia Y. Lee (Centre Médical de l'Université de l'Arkansas, Etats-Unis). Il s'agit du congelat d'origine.

### Préparation des congelats

Deux types de congelats ont été préparés. Le congélat (A) est issu directement du congelat d'origine par amplification sur milieu columbia et contient comme lui des matières d'origine animale. Un second congelat (B) a été dérivé du congélat d'origine par clonage et passages répétés sur milieu dépourvu de matières d'origine animale.

Une pré-culture en milieu TSB (bouillon trypticase soja) est ensemencée à partir de 100 µl de congelat initial et placée sous agitation 18 heures à 37°C. Une culture de 400 ml est ensuite ensemencée à partir de la pré-culture. Au bout de 5 heures sous agitation à 37°C, 200 ml de culture sont ajoutés à 50 ml de glycérol filtré sur 0,22 µm. La répartition des congelats et réalisée sous 100 tubes de 100 µl et 100 tubes de 1 ml puis stockage à -80°C.

### Préparation des congelats des lots de semence A et B

### Préparation des pré-congelats

Des étapes de clonage sont réalisées sur milieu M0 non inducteur. A partir d'un aliquot de 100 µl de la souche CYL1892 d'origine, un isolement est réalisé sur milieu M0 non inducteur. A partir de cet isolement, deux colonies sont piquées, reprises par 50 µl d'H₂O sans nucléase puis ensemencées à nouveau en colonies isolées. Au bout de trois passages, une colonie par boîte est reprise puis ensemencée en nappe (un volume de reprise de colonie est prélevé en parallèle pour réaliser un contrôle par PCR). La nappe ainsi obtenue est ensuite utilisée pour l'ensemencement d'une culture de 5 heures de milieu M0 liquide inducteur (50 ml). A l'issue de la culture, la densité optique à 680 nm est mesurée, puis 40 ml de culture sont ajoutés à 10 ml de glycérol codifié et filtré 0,22 µm. La répartition est réalisée sous 45 x1 ml et stockage à -80°C.

### Culture et congélation du congelat B

Des dilutions en séries sont réalisées dans le but d'éliminer tous les composants d'origine animale éventuellement présents dans le congelat d'origine. Le milieu M0 non inducteur est utilisé lors des passages.

Une dilution au 100^{ème} est réalisée sur le pré-congelat B de départ avant le premier passage sur milieu gélosé. Des dilutions en séries (10⁻⁵) sont ensuite réalisées entre chaque passage (passages 2 à 4). Au total, quatre passages sur milieu gélosé ont été effectués, avec un facteur de dilution total de 10⁻¹⁷. La nappe correspondant à la dernière dilution effectuée est utilisée pour l'ensemencement d'une culture de 400 ml de milieu M0 liquide inducteur. La culture est stoppée en fin de phase exponentielle après 5 heures de culture à 37°C sous agitation. Des mesures de DO₆₈₀ₙₘ sont réalisées tout au long de la cinétique de croissance. La culture est ensuite supplémentée de glycérol à 20 % final puis répartie en 6 flacons Schott maintenus dans la glace jusqu'à la répartition finale en tubes. La répartition est effectuée sous 1 ml en tubes Nunc et également sous 5 ml en tube Falcon 15 ml. Les ampoules sont ensuite placées à -80°C.

### Contrôles réalisés

### Viabilité

Des numérotations sur chaque flacon de répartition sont réalisées au moment de la répartition des congelats B avant congélation selon le protocole :
- dilution en PBS des congelats en séries de 10 en 10 jusqu'à la dilution 10⁻⁹,
- dépôt de 6 gouttes de 25 µl des dilutions 10⁻³ à 10⁻⁹ sur des boîtes 24x24 Nunc de gélose Columbia 2 % NaCl,
- incubation à 37°C pendant 18 heures,
- comptage des colonies dans la gamme de dilution la plus lisible et calcul de moyenne sur les flacons de répartition.

Des contrôles de viabilité ont ensuite été réalisés suivant la même technique de numération sur plusieurs congelats correspondant à chaque flacon de répartition suivant le planning : J+11 jours, J+1 mois, J+3 mois, J+6 mois, J+1 an.

### Identité

L'identité est contrôlée à partir d'un congelat à l'aide des tests biochimiques de la galerie ApiStaph® et d'un test de résistance à la lysostaphine. Ces tests ont été réalisés à partir d'une culture de 18 heures à 37°C sur gélose Columbia 2 % NaCl et selon les indications données par le fabricant.

D'autre part, un typage capsulaire par amplification PCR multiplex T5/T8 est réalisé à partir d'un isolement sur gélose Columbia 2 % NaCl. Les souches Reynolds et Becker sont utilisées comme témoins pour les amplifications T5 et T8.

Un test PCR de stabilité génétique cap1 utilisant les oligonucléotides Ppa1Ncol et Ppa1r (décrit par Luong et al dans Infect. Immun. 2002 ; 70(7) :3389-95) est également réalisé : un isolement est réalisé sur gélose Columbia 2 % NaCl et incubé pendant 24 heures à 37°C. Une colonie est ensuite récupérée pour effectuer un second passage sur gélose Columbia 2 % NaCl. Quatre passages successifs sont ainsi réalisés. L'ADN des colonies est extrait pour vérifier par PCR la présence du promoteur cap1. La souche CYL770 est utilisée comme témoin pour l'amplification du promoteur cap1.

Un contrôle d'identité des polysaccharides capsulaires des souches de T5 et T8 est réalisé par test d'agglutination sur lame à l'aide d'anticorps monoclonaux spécifiques anti-PS5 (IgM) clone J1 et anti-PS8 (IgG1) clone K8-24 obtenus à partir d'hybridomes fournis par l'Institut pasteur qui ont été produits par des procédés classiques bien connus de l'homme de l'art. Les réactions d'agglutination sur lame sont réalisées à partir de 50 µl de suspension bactérienne ajustée à DO₆₈₀ₙₘ=5.

### Evaluation de la productivité par ELISA

### Test de productivité

Une pré-culture en milieu M0 liquide inducteur est ensemencée à partir des congelats à tester. Une culture de 400 ml en milieu inducteur M1 est ensuite ensemencée et placée sous agitation à 37°C pendant 72 heures. En fin de culture, un volume de 50 ml de culture est prélevé et centrifugé 30 minutes à 3500 rpm à +4°C. Les polysaccharides capsulaires sont ensuite extraits puis dosés par ELISA.

### Test de stabilité

Des cultures successives en milieu M1 sont réalisées de façon à générer un nombre de générations proche de ce qui pourrait être obtenu dans un fermenteur de 1 000 litres. Pour cela, on ensemence 10 ml de M1 avec une ampoule d'un congelat B puis, après 16h de culture à 37°C, on réalise 8 cultures successives en ensemençant 10 ml de milieu M1 à DO=0,2 et en cultivant respectivement pendant 4h, 4h, 16h, 4h, 4h, 16h, 4h et 4h à 37°C. A partir de la dernière culture on ensemence 150 µl sur un milieu M0 gélosé inducteur pour réaliser une préculture à 37°C pendant 16h, puis on ensemence 400 ml de M1 à DO=0,2 et on cultive pendant 72h à 37°C. A la suite du dernier passage sur milieu M1, une mesure de productivité est réalisée comme précédemment et comparée à la productivité obtenue pour un congelat de référence dans les mêmes conditions de culture.

### Extraction autoclave et préparation des échantillons

En fin de culture, les suspensions sont centrifugées 30 minutes à 3500 rpm à +4°C, les surnageants de culture sont séparés et stockés à +4°C. Les culots sont extraits grâce à deux cycles d'autoclave successifs. Chaque cycle se compose d'une reprise des culots dans 5 ml de PBS suivi d'un cycle d'autoclave 60 minutes à 121°C. Un test de mortalité sur les culots autoclavés est réalisé par ensemencement sur gélose Columbia 2% NaCl. Les culots autoclavés sont ensuite centrifugés 30 minutes 25 000 x g à +4°C et l'extrait (surnageant) récupéré puis stocké à +4°C. Le culot est ensuite à nouveau repris dans 5 ml de PBS et un second cycle d'autoclave est engagé. En fin de deuxième cycle, les deux extraits sont regroupés. Les extraits et surnageants de culture sont traités à la protéinase K (50 µg/ml) pendant 1 heure à 37°C puis la protéinase K est inactivée par incubation 30 minutes à 80°C. Un test de mortalité est ensuite réalisé sur les surnageants de culture traités.

### Dosage ELISA PS5

Les échantillons traités à la protéinase K (extraits obtenus après autoclave et/ou surnageants de culture traités) sont dosés en ELISA sandwich. Un double titrage permet de s'assurer de la fiabilité des données. Le principe consiste à capturer l'antigène (PS5) entre deux anticorps pour le quantifier. Afin d'éviter toute interférence et obtenir la mesure la plus précise possible, l'un des deux anticorps doit être parfaitement spécifique (anticorps monoclonal anti-PS5 utilisé en adsorption). Un sérum polyclonal de lapin adsorbé est ensuite utilisé en temps qu'anticorps de détection. Un anticorps secondaire conjugué à la peroxydase permettra la révélation. Lors de l'ELISA, une courbe standard est effectuée avec du PS5 purifié, utilisé à différentes concentration pour quantifier l'antigène. Un contrôle est également introduit, il s'agit de PS5 purifié dont la concentration est connue et établie par mesure physicochimique à 78 ug/ml. Dans l'étape finale, un substrat chromogène le tétraméthyl benzidine (TMB) est ajouté. Celui-ci est dégradé par la peroxydase en un produit coloré bleu. Un acide est ajouté après 15 minutes d'incubation à 20°C et à l'obscurité, ce qui provoque l'arrêt de la réaction et transforme le produit coloré bleu en jaune. La densité optique est mesurée à 450 nm, elle est proportionnelle à la quantité d'anticorps fixé.

### Résultats

### Congelats A CYL 1892

Après 11 jours de congélation, les résultats de numération de CYL1892 sont stables. Quatre passages successifs ont été réalisés. Au total, 16 colonies distinctes ont été extraites et analysées en PCR. Toutes les colonies testées à chaque passage possèdent bien le locus *cap* de type 5 et la présence du promoteur *capl* a été démontrée pour toutes les colonies également.

### Congelats B CYL1892

Après 11 jours de congélation, les résultats de numération de CYL1892 sont stables (2,3x10⁹ CFU/ml en moyenne).

### Galerie Api

Une identification correcte de *S. aureus* est obtenue suivant l'analyse des caractères biochimiques selon les indications du fabricant.

### Profil PCR

La stabilité génétique du pré-congelat a été suivie lors des différentes étapes de clonage. On note la présence d'une amplification spécifique de *cap5* à environ 500 pb et du promoteur *cap1* à 200 pb. Les dix colonies testées sont issues du pré-congelat B ayant servi à l'ensemencement de la culture pour la réalisation du congelat B. On observe la stabilité génétique des loci *cap5* et promoteur *cap1.*

Vingt colonies isolées provenant du congelat B CYL1892 ont été testées. Les amplifications à environ 500 pb et 200 pb illustrent la stabilité génétique des loci *cap5* et promoteur *cap1.*

### Test d'agglutination

Seule une agglutination avec l'anticorps monoclonal anti-PS5 est observée.

### Productivité des congelats A et B CYL 1892 (Figure 1)

Des mesures de productivité ont été réalisées plusieurs fois en parallèle avec des références de congelats et des congelats de la souche sauvage. Comme pour les souches recombinantes, deux types de congelats ont été préparés pour les souches sauvages. Un congelat Reynolds A a été obtenu à partir de la souche d'origine par un faible nombre de passages au laboratoire en bouillon columbia 2% NaCl. Ce congelat A renferme des matières d'origine animale. Un second congelat (congelat Reynolds B), dépourvu de matière d'origine animale a été obtenu par passages successifs de la souche d'origine sur milieux M0. Les congelats Reynolds B ont été préparés en milieu M0 liquide inducteur après avoir subi des passages en milieu M0 non inducteur.

On observe que la souche recombinante CYL1892 produit en moyenne 3,5 fois plus de PS5 dans le surnageant que dans le culot. La quantité totale ainsi produite représente environ 10 fois plus de PS5 par rapport à la souche sauvage Reynolds (300 µg/ml et 30 µg/ml, respectivement) après culture en milieu inducteur liquide M1. La différence est très significative dans les surnageants. On observe une production de PS5 environ 50 fois plus importante pour la souche CYL1892 par rapport à la souche sauvage Reynolds. La différence est beaucoup moins importante en ce qui concerne les culots. En effet, on observe une différence de productivité PS5 d'un facteur 2 pour la souche CYL1982 par rapport à la souche Reynolds. On constate que le congelat CYL1892 B produit une quantité équivalente de PS5 par rapport au congelat A.

### Stabilité des congelats A et B CYL 1892 (Figure 1)

Des mesures de productivité ont été réalisées après un test de stabilité sur milieu M1 (environ 40 générations effectuées). La productivité observée dans les culots et surnageants après plusieurs passages en milieu M1 est équivalente à celle obtenue sans passage pour le congelat B CYL1892 comme pour le congelat A. Une baisse de productivité de 20 à 50 % est observée pour les congelats B de la souche sauvage Reynolds.

La caractérisation de la souche recombinante *S. aureus* de type 5 CYL1892 a permis de constater qu'un gain de productivité en polysaccharides capsulaires est obtenu par rapport à l'utilisation de la souche sauvage Reynolds. De façon surprenante, la différence de productivité a été essentiellement observée dans le surnageant de culture (environ 50x, soit 200 µg/ml en moyenne) et reste stable après passages successifs en milieu inducteur. Ces résultats montrent que la construction utilisée pour la réalisation de cette souche recombinante est stable. Les congelats A et B ont donc été réalisés à partir de populations homogènes.

### Exemple 2 : Caractérisation du polysaccharide capsulaire de type 5 produit par la souche CYL1892, et du polysaccharide capsulaire de type 8 produit par la souche CYL770

Les rPS T8 et T5 isolés et purifiés à partir des souches CYL770 et CYL1892 respectivement ont été caractérisés par les analyses décrites ci-dessous.

Une analyse par résonance magnétique du proton à une dimension de rPS5 et rPS8 purifiés a montré que ces polysaccharides ont la même structure primaire que celle déjà publiée (C Jones. Carbohydr. Res. 2005, 340, 1097-1106). Cette analyse permet de plus de montrer directement que le rPS5 possède un taux de N- et O- acétylation supérieur à 70 %. Pour le polysaccharide de type 8, la même analyse, effectuée après dé-O-acétylation de l'antigène à l'aide de NaOD rajouté directement dans le tube RMN, a montré que le degré de N- et O- acétylation est supérieur à 70%. Pour le rPS5 et le rPS8, aucune éventuelle impureté de nature glycosidique non-identifiée ou identifiée (comme du peptidoglycane, de l'acide téichoïque, de l'acide lipotéichoïque, et l'antigène 336), susceptible de provenir de *S. aureus,* n'a été détectée par analyse RMN. Les spectres RMN des rPS8 et rPS5 purifiés à partir des souches CYL770 et CYL1892 respectivement sont donnés aux figures 5 et 6.

Une chromatographie haute performance d'exclusion stérique a été effectuée en utilisant comme détecteurs en ligne un détecteur à diffusion de lumière, un viscosimètre et un réfractomètre. Cette analyse a permis d'évaluer les poids moléculaires moyens des rPS8 et rPS5 à 250 kDa et 150 kDa, respectivement. Ces valeurs de poids moléculaire sont plus élevées que celles des polysaccharides capsulaires purifiés à partir de la souche S. *aureus* Becker (type 8) ou Reynolds (type 5) cultivée avec le même milieu et dans les mêmes conditions de culture et purifiées à partir du culot cellulaire (poids moléculaire moyen estimé à 150 kDa et 50 kDa, respectivement).

### Exemple 3 Préparation d'un sérum polyclonal anti-CYL770 adsorbé sur germes chimiquement acapsulés et évaluation de sa réactivité croisée sur différentes souches de S. aureus de type 8

Une culture de la souche recombinante de type 8 CYL770 a été réalisée en milieu inducteur M1. Les germes issus de cette culture ont été formolés pour servir à un protocole d'immunisation chez le lapin selon le principe décrit dans la littérature (Karakawa et al., 1985 J Clin Microbiol 22: 445-447). Le sérum hyperimmun obtenu contient des anticorps spécifiques des polysaccharides capsulaires de type 8, mais également des anticorps dirigés contre d'autres épitopes pouvant être communs entre les différentes souches. Ce sérum a donc été adsorbé plusieurs fois sur des culots à 10¹¹ CFU de la souche autologue rendue acapsulée chimiquement de façon à éliminer les anticorps non spécifiques du polysaccharide capsulaire de type 8. Le réactif ainsi obtenu a été ensuite évalué par technique d'agglutination sur germes entiers tryspsinés afin de s'affranchir de la fixation non spécifique des anticorps via la protéine A, sur les germes issus de cultures de la souche recombinante CYL770 en milieu inducteur M1. Puis le sérum anti-CYL770 a été testé dans les mêmes conditions sur des germes entiers trypsinés des souches sauvages de type 8 (Becker et Wright) issus de cultures solides et liquides de différents niveaux d'induction de polysaccharides capsulaires.

### Préparation des germes CYL 770 pour immunisation

Une pré-culture est réalisée en erlenmeyer en ensemençant une ampoule (1 ml) de congelat A de CYL770 dans 9ml de milieu M0 liquide inducteur. Les pré-cultures sont placées sous agitation x100 à 37°C pendant 18h à 20h. La souche CYL770 est ensuite cultivée en erlenmeyer dans 100 ml M1 en ensemençant à DO₆₈₀ₙₘ=0.2 à partir de la pré-culture réalisée en M0 liquide inducteur. L'erlenmeyer est alors placé sous agitation x100 à 37°C pendant 48h. La culture est ensuite centrifugée, le culot est repris par 20ml de PBS (solution mère) et la DO₆₈₀ₙₘ est mesurée.

Un volume de solution mère est prélevé afin de réaliser la numération avant formulation. A partir du prélèvement de solution mère CYL770, des dilutions de raison 10 sont réalisées en plaque 24 puits de 10⁻¹ à 10⁻⁹. Six gouttes de 25µl de chaque dilution sont ensemencées sur gélose Columbia 2% NaCl et incubées pendant 18 à 20h à 37°C. Les CFU sont dénombrées aux dilutions où cela est possible et le nombre de CFU/mL (X) est déterminé selon la formule : X = (moyenne des CFU par goutte x 40) / dilution de lecture. La détermination de l'équivalence DO/CFU (Y) est effectuée comme suit : pour DO₆₈₀ₙₘ = 1, Y = (X / DO₆₈₀ₙₘ solution mère).

La fixation au formol est réalisée pendant 15h en diluant un volume de solution mère CYL770 dans du PBS-3% formol de manière à obtenir 80ml de suspension à DO₆₈₀ₙₘ=1,2, selon le protocole décrit par (Karakawa et al., 1985 J Clin Microbiol 22: 445-447). Après 15h de traitement, un test de mortalité est effectué par amplification d'1mL de suspension formolée ensemencée dans 9mL de TSB et placé sous agitation x100 à 37°C pendant 18 à 20h, puis ensemencement d'un test de mortalité sur gélose Columbia 2% NaCl par étalement de 150 µl de la culture amplifiée en nappe et incubation pendant 18 à 20h à 37°C.

Les germes traités au formol subissent ensuite trois lavages successifs avec du tampon phosphate (PBS) 1X concentré pH 7 afin d'éliminer le formol centrifugation 30min à 3500rpm +4°C (2x 40mL), lavage 2x 40mL, centrifugation 30min à 3500rpm +4°C, lavage 2x 30ml, centrifugation 30min à 3500rpm +4°C, lavage 2x 20ml (pool), centrifugation 30min à 3500rpm +4°C, et reprise des germes en PBS. La suspension de germes ainsi traitée peut être stockée à + 4°C.

Les germes formolés sont ajustés et aliquotés sous 4ml en flacons de 5mL stériles. L'ajustement des suspensions de germes formolés est réalisé par rapport aux DO₆₈₀ₙₘ, équivalentes aux concentrations de germes requises :
2x 4ml à 6x10⁷ CFU/ml (injections j₂ + j₄)
4x 4ml à 1,2x10⁸ CFU/ml (injections j₀+j₇+j₉+j₁₁)
3x 4ml à 1,8x10⁸ CFU/ml (injections j₁₄ + j₁₆ + j₁₈)
3x 4ml à 2,4x10⁸ CFU/ml (injections j₂₁ + j₂₃ +j₂₅)

Les bouchons sont placés stérilement puis les flacons sont sertis à l'aide d'une pince. Les suspensions sont stockées à +4°C une semaine.

### Préparation des germes chimiquement acapsulés pour adsorption

A partir d'une ampoule du congelat A (1 ml) de la souche CYL770, une préculture de 50ml de milieu Columbia est ensemencée et incubée à 37°C sous agitation pendant 18h. A partir de la préculture, une culture de 400ml de milieu Columbia est ensemencée à DO₆₈₀ₙₘ initiale de 0,2 et incubée à 37°C sous agitation pendant 24h. La culture est ensuite centrifugée à 3500rpm pendant 30min à +4°C et le surnageant est éliminé. Le culot est ensuite repris par 8ml de PBS et une mesure de DO est réalisée : DO₆₈₀ₙₘ=178.

Le traitement chimique à appliquer aux germes afin d'éliminer les polysaccharides capsulaires a été décrit par Karakawa *et al.* (1985 Karakawa et al., 1985 J Clin Microbiol 22: 445-447). Le traitement suivant est réalisé en double : 4ml de suspension bactérienne à DO=178 sont transférés dans un flacon Schott en verre de 250ml, 30ml de glycine 0,2M HCl 0,14M pH 2 sont ajoutés et le mélange est incubé 20min exactement à 100°C (bain-marie). La suspension est transférée en tubes Falcon 50ml, centrifugée à 3500 rpm pendant 30min à +4°C et le surnageant éliminé. Les culots sont lavés 3 fois avec 40ml de PBS. Les culots sont ensuite repris et poolés avec 30ml de PBS et une mesure de DO est réalisée pour déterminer le volume de répartition par tube de façon à avoir un total de 10¹¹ CFU. Après centrifugation à 3500 rpm pendant 30min à +4°C et élimination du surnageant, les culots secs sont stockés à -20°C jusqu'à utilisation.

### Adsorption du sérum sur germes chimiquement acapsulés

Deux ml de sérum polyclonal anti-CYL770 ont été utilisés pour le protocole d'adsorption. Un culot bactérien de 10¹¹ CFU permet de réaliser un cycle d'adsorption pour 2ml de sérum. Au total, 5 cycles d'adsorption ont été réalisés, chaque cycle se composant de la façon suivante : déposer 2ml de sérum décomplémenté anti-CYL770 sur un culot de germes chimiquement acapsulés, resuspendre doucement, placer sur un rotateur pour tubes à +4°C sur la journée ou sur la nuit, centrifuger à 3500rpm pendant 30min à +4°C, récupérer le sérum adsorbé, déposer le sérum sur un nouveau culot de germes chimiquement acapsulés et reprendre le protocole. Après le cinquième cycle, le sérum adsorbé est récupéré et filtré sur membrane Millipore 0,22µm puis stocké à +4°C jusqu'à son évaluation par test d'agglutination sur lame.

### Test du sérum adsorbé en agglutination

Le test d'agglutination sur lame du sérum polyclonal anti-CYL770 adsorbé sur germes chimiquement acapsulés a été réalisé sur des germes trypsinés issus de cultures sur différents milieux solides et liquides des souches de S. *aureus* de type 8 (Becker, Wright et CYL770). Une culture en milieu solide non inducteur de PS de la souche de *S. aureus* de type 5 Reynolds a également été testée en tant que témoin.

Pour préparer les germes trypsinés, des cultures en milieu solide M0 gélosé inducteur (pour les souches de type 8) et Columbia 2% NaCl (pour la souche Reynolds) ont été réalisées de la façon suivante : 900µl de PBS sont ajoutés à 100µl de congelat des souches Becker, Wright et Reynolds et 150µl de suspension bactérienne sont étalés en nappe sur une boîte de pétri et incubés à 37°C pendant 18h (37°C + 5% CO₂ pour la souche Becker). Les nappes sont ensuite reprises en PBS et les suspensions sont ajustées à DO₆₈₀ₙₘ=5.

Pour les souches de type 8 Becker et Wright, des cultures liquides de 50ml en milieu TSB et en milieu M1 ont été réalisées à partir de précultures solides en milieu TSB et M0 gélosé inducteur respectivement. Les cultures solides ont été ensemencées directement à partir d'un congelat comme décrit précédemment. Les nappes des cultures solides ont été resuspendues en PBS et ont servi à ensemencer les cultures liquides à DO₆₈₀ₙₘ, initiale de 0,2.

Pour la souche recombinante CYL770, une culture liquide de 50ml en milieu M1 a été réalisée. Une préculture de 10ml en milieu M0 liquide inducteur a été directement ensemencée à partir d'un congelat de 1ml. Après 18h d'incubation à 37°C sous agitation, la culture en milieu M1 a été ensemencée à DO₆₈₀ₙₘ initiale de 0,2 à partir de la préculture liquide. Les cultures liquides ont été incubées pendant 24h à 37°C sous agitation. Après 24h de culture, la DO₆₈₀ₙₘ a été mesurée et les cultures ont été centrifugées à 3500 rpm pendant 30min à +4°C. Les culots ont été repris dans un volume de PBS nécessaire afin d'ajuster les suspensions à DO₆₈₀ₙₘ, = 5.

Les suspensions bactériennes issues des cultures solides et liquides et ajustées à DO₆₈₀ₙₘ = 5 sont traitées à la trypsine de façon à éliminer toute trace gênante de protéine A. Pour cela, à partir d'une aliquote de suspension bactérienne de 5ml, 100µl de trypsine à 50mg/ml ont été ajoutés et le mélange incubé 1 h à 37°C sur rotateur puis centrifugé à 3500rpm pendant 30min à +4°C. Les culots ont été lavés 3 fois avec 5ml de PBS et repris avec le volume nécessaire de PBS afin d'ajuster les suspensions à DO₆₈₀ₙₘ = 5. Les suspensions sont stockées à +4°C jusqu'à utilisation.

Les tests d'agglutination ont été réalisés avec 50µl de germes frais trypsinés à DO₆₈₀ₙₘ = 5. Les germes ont été étalés sur la lame de manière à recouvrir totalement le cercle de verre délimité destiné à l'agglutination sans déborder vers l'extérieur. Un témoin germe a tout d'abord été réalisé avec 50µl de suspension de germes sur lesquels 5µl de PBS ont été déposés. Le témoin est validé en l'absence d'agglutination après 5 minutes de mise en contact. Un volume de 5µl de sérum polyclonal anti-CYL770 adsorbé sur germes chimiquement décapsulés a été utilisé pour les tests d'agglutination. Après dépôt du sérum sur la lame dans les trois puits, la lame a été agitée manuellement d'un mouvement circulaire au-dessus d'un miroir de Kahn (miroir concave). Cette étape a été chronométrée à partir de l'ajout du sérum dans le premier puits.

### Résultats

La souche CYL770 est cultivée en milieu M1 pendant 48h, elle est ensuite fixée au formol. La mortalité directe ainsi que la mortalité après amplification est vérifiée. La suspension est alors ajustée aux concentrations requises (Tableau 2) et répartie en flacons stériles. Les flacons sont ensuite sertis et utilisés pour l'immunisation selon le protocole décrit dans la littérature (Karakawa et al., 1985 J Clin Microbiol 22: 445-447).

**Tableau 2 : Concentration des germes CYL770 formolés pour immunisation**

| | Equivalence DO/CFU | Concentrations théoriques | | | Solutions germes formolés | |
|---|---|---|---|---|---|---|
| | CFU/ml pour DO₆₈₀=1 | Volumes à préparer | Concentrations finales désirées | DO₆₈₀ correspondantes | DO₆₈₀ mesurées | Concentrations |
| CYL770 Bouillon M1 | 1,06 x10⁹ | 10 | 6,0 x10⁷ | 0,038 | 0,040 | 6,2 x10⁷ |
| | | 20 | 1,2 x10⁸ | 0,077 | 0,079 | 1,2 x10⁸ |
| | | 15 | 1,8 x10⁸ | 0,115 | 0,115 | 1,8 x10⁸ |
| | | 5 | 2,4 x10⁸ | 0,154 | 0,159 | 2,5 x10⁸ |

Une fois produit, le sérum est adsorbé sur germes autologues chimiquement acapsulés puis évalué contre les souches de type 8 en agglutination sur lame (Tableau 3).

**Tableau 3 : Evaluation du sérum anti-CYL770 adsorbé en agglutination sur lame**

| | Becker | | | CYL770 | Wright | | | Reynolds |
|---|---|---|---|---|---|---|---|---|
| | M0 gélosé inducteur | M1 | TSB | M1 | M0 gélosé inducteur | M1 | TSB | Agar Columbia |
| PBS | - | - | - | - | - | - | - | - |
| Sérum anti-CYL770 adsorbé | + + + | + + + | - | + + + | + + | + + + | + + | - |
| Mab-PS8 | ++(+) | +++ | - | ++++ | ++++ | ++++ | ++ | - |

La lecture du test d'agglutination a été réalisée de la façon suivante :
- ++++ agglutination totale observée en 0 à 30 sec
- +++ agglutination totale observée en 30 sec à 1 min
- + + agglutination totale observée en 1 à 3 min
- + agglutination totale observée en 3 à 5 min
- +/- agglutination très lente et totale observée en 10 min
- aucune agglutination observée

On observe une agglutination des souches Becker (excepté en milieu TSB), Wright et CYL770 avec le sérum anti-CYL770 adsorbé. Par ailleurs, ce sérum ne permet pas l'agglutination des germes témoins Reynolds (type 5) cultivés en milieu de référence gélose Columbia 2% NaCl, ni des germes Becker cultivés en milieu TSB non inducteur de PS8 démontrant ainsi sa spécificité.

Le sérum anti-CYL770 adsorbé agglutine d'autant plus rapidement les germes lorsque ceux-ci sont porteurs de capsule de type 8. On observe le même profil d'agglutination avec l'anticorps monoclonal anti-PS8 (Mab-PS8).

Le sérum anti-CYL770 adsorbé n'a donc montré aucune différence de reconnaissance sur les trois souches testées lorsque celles-ci sont placées dans des conditions inductrices de capsule. L'agglutination étant liée à la présence d'antigènes capsulaires, aucune différence entre les polysaccharides capsulaires de type 8 produits par ces différentes souches n'est donc à noter.

Il apparaît donc que le sérum anti-CYL770 adsorbé ainsi produit permet une réactivité croisée sur les germes sauvages présentant une capsule de type 8.

### Exemple 4 : Génération d'anticorps polyclonaux anti-PS8 chez le lapin à partir de la souche CYL770

Pour caractériser la nature du PS8 produit par la souche CYL770, des anticorps polyclonaux anti-PS8 ont été générés par l'immunisation de lapins avec des germes entiers inactivés de la souche CYL770 cultivés en milieu M1, en s'inspirant du protocole précédemment décrit par Karakawa et collaborateurs (Karakawa et al., 1985; J. Clin. Microbiol., 22 : 445-447).

### Protocole d'immunisation

**Injections :** Trois lapins femelles New Zealand White (ESD - Charles River Laboratories, St Germain-sur-l'Arbresle, France) de 2,5 kg sont injectés 3 fois par semaine pendant 4 semaines avec différentes concentrations de germes entiers inactivés de la souche CYL770 cultivés en milieu M1 et dialysés contre du PBS. La première injection (J0) est effectuée par voie sous-cutanée dans la ceinture scapulaire pour éviter les chocs septiques. Toutes les injections suivantes sont réalisées par voie intraveineuse dans la veine marginale de l'oreille. Les concentrations et les volumes utilisés pour chacune des injections sont décrits ci-dessous.

**Tableau 4 : Description des groupes**

| Groupes | Antigène | Tampon de dilution | Injection | |
|---|---|---|---|---|
| | | | Route | Volume |
| 1 : 1 lapin | PBS | PBS 1X, pH 7,2 | SC pour la première injection IV pour toutes les autres injections | 1 ml |
| 2 : 2 lapins | CYL770 en milieu M1 0,6 à 2,4x10⁸ CFU/lapin | PBS 1X, pH 7,2 | SC pour la première injection IV pour toutes les autres injections | 1 ml |

| | | | | |
|---|---|---|---|---|
| Composition du PBS 1X pH 7,2: 136 mM Nacl; 2,7 mM KCl, 8 mM Na₂HPO₄; 7,5 mM KH₂PO₄ | | | | |

**Prélèvement de sang sur les animaux pré-immuns :** Cinq à 10 ml de sang sont collectés dans des tubes Vacutainer^{™} avant la première immunisation (J-1) pour chaque lapin, localement anesthésiés à la Xylocaïne, dans l'artère médiane de l'oreille. Les échantillons sont laissés à exsuder pendant 3h à 4h à température ambiante (20-22°C) puis centrifugés à 4°C pendant 20 min à 1500 g

**Prélèvement de sang par exsanguination :** Tous les animaux sont prélevés par ponction cardiaque sous anesthésie générale, 28 jours après la première injection. Entre 50ml et 80 ml de sang sont prélevés par animal et collectés dans des tubes Falcon^{™} 50 ml stériles.

Tous les sérums sont conservés à -20°C jusqu'à utilisation

**Tableau 5 : Schéma d'immunisation**

| Semaine | Jour | Injections | Interventions |
|---|---|---|---|
| | J-1 | - | Prélèvements de sang (10 ml /animal) |
| 1 | J0 | 1 ml, *S.C.* (1,2x10⁸ CFU) | Sensibilisation (1^{ère} injection en SC) |
| | J2 | 1 ml, *I.V*. (6x10⁷ CFU) | Amplification |
| | J4 | 1 ml, *I.V*. (6x10⁷ CFU) | Amplification |
| 2 | J7 | 1 ml, *I.V.* (1,2x10⁸ CFU) | Amplification |
| | J9 | 1 ml*, I.V.* (1,2x10⁸ CFU) | Amplification |
| | J11 | 1 ml*, I.V.* (1,2x10⁸ CFU) | Amplification |
| 3 | J14 | 1 *ml, I.V.* (1,8x10⁸ CFU) | Amplification |
| | J16 | 1 ml*, I.V.* (1,8x10⁸ CFU) | Amplification |
| | J18 | 1 ml, *I.V.* (1,8x10⁸ CFU) | Amplification |
| 4 | J21 | 1 *ml, I.V.* (2,4x10⁸ CFU) | Amplification |
| | J23 | 1 ml*, I.V.* (2,4x10⁸ CFU) | Amplification |
| | J25 | 1 ml, *I.V.* (2,4x10⁸ CFU) | Amplification |
| 5 | J28 | — | Exsanguination (50 ml à 80 ml / lapin) |

### Analyses des réponses anticorps anti-PS8

Les sérums polyclonaux de lapins sont évalués pour leur spécificité vis à vis du PS8 par un test ELISA. Des plaques ELISA de 96 puits (flat-bottom microplates; Immunosorp Microwell; Nunc; Roskilde, Danemark) sont incubées pendant une nuit à température ambiante (20-22°C) en présence de 1 µg PS8 purifié par ml dans du PBS 1 X pH 7.2 (100 µl / puits). Les plaques sont lavées 4 fois avec 300 µl / puits de PBS / 0.05 % Tween 20 (PBS-Tween) à l'aide du laveur de plaques automatiques Titertek M96V Washer puis saturées pendant 1h à 37°C avec 250 µl / puits de PBS-Tween / 1% BSA. Les plaques sont ensuite lavées 4 fois selon la méthode décrite précédemment. 100 µl par puits de chacun des sérums à tester à différentes concentrations sont déposés dans la plaque, les dilutions successives de raison trois sont réalisées au sein même de la plaque (dilution en colonne) à l'aide d'une pipette multicanaux dans du PBS-Tween 1 % BSA. Les plaques sont incubées pendant 1h30 à 37°C puis lavées 4 fois avec du PBS-Tween.

Un conjugué anti-IgG de lapin conjugué à la HRP (Southern Biochemicals Inc. Birmingham) est dilué au 1/12000 dans du PBS-Tween / 1% BSA et 100 µl de la solution de conjugué sont ajoutés par puits. Les plaques sont incubées pendant 1h30à 37°C puis lavées 4 fois avec du PBS-Tween. Pour la révélation, 100 µl de la solution de substrat TMB (TEBU Bio-laboratories) prête à l'emploi sont ajoutés par puits et les plaques sont incubées pendant 15 min à température ambiante (20-22°C) et à l'obscurité. La réaction enzymatique due à la peroxydase est stoppée par l'ajout de 100 µl de H₃PO₄ 1M par puits. La densité optique (DO) de chacun des puits est mesurée à 450 nm à l'aide d'un lecteur de plaque automatique (Versamax). Le bruit de fond (valeur moyenne sur 4 puits blancs) est soustrait aux valeurs de DO mesurées.

Les titres spécifiques anti-PS8 des anticorps polyclonaux correspondent à l'inverse de la moyenne arithmétique des dilutions observées pour deux puits à 450 nm, par rapport à un sérum de référence.

**Tableau 6 : Titres IgG anti-PS8**

| | | Sérums | |
|---|---|---|---|
| | Becker M1 Contrôle positif | CYL770 M1 | CYL770 M1 |
| Titres IgG anti-PS8 | 30 000 | 8500 | 5000 |

### Exemple 5 : Production de conjugués

### 5.1- Production de conjugués de type 8 (STAPH8-rEPA)

### Dépolymérisation du polysaccharide de type 8 purifié à partir de la souche CYL770

Avant sa conjugaison, l'antigène rPS8 purifié à partir de la souche CYL770 est dépolymérisé selon le principe décrit dans le brevet US 6,045,805. L'antigène rPS8 est préparé à 2 mg/ml. A 20 ml de cette solution à température ambiante, sont ajoutés 110 µl d'acide ascorbique 200 mM, 11 µl de CuSO4 20 mM et 11 µl de FeS04 20 mM. La réaction est poursuivie sous agitation pendant 80 min à température ambiante. La réaction de dépolymérisation est stoppée par ajout de 2 ml d'une solution de TRIS 2 M à pH 7,0. Les réactifs de dépolymérisation sont éliminés par dialyse contre de l'eau puis le rPS8 dépolymérisé est lyophilisé. La masse moléculaire moyenne du rPS8 dépolymérisé a été estimée proche de 50 kDa par analyse HPSEC/LS/RI/Visc.

### Activation de l'antigène

40 mg d'antigène (dépolymérisé pour le polysaccharide purifié à partir de la souche CYL770) ont été dissous dans 8 ml de NaCl 200 mM et d'acide adipique dihydrazide (ADH) 125 mM. Le pH est ajusté à 4,9 et de l'éthyldiméthylaminopropylcarbodiimide (EDAC) est ajouté à une concentration finale de 25 mM. Pendant le déroulement de l'activation qui a duré 90 min à température ambiante, le pH est constamment ajusté à une valeur de 4,9 par ajout d'une solution diluée de HCl. La réaction est stoppée par neutralisation du pH. L'antigène activé est ensuite dialysé contre NaCl 500 Mm puis contre l'eau. L'antigène activé et dialysé est alors lyophilisé. Le pourcentage de fonctionnalisation a été estimé à 3,7 et 4,3 (p/p) pour le polysaccharide purifié à partir de la souche Becker et pour le polysaccharide purifié et dépolymérisé à partir de la souche CYL770, respectivement.

### Conjugaison de l'antigène

Une solution de 10 ml contenant 20 mg de l'antigène activé (et dépolymérisé pour l'antigène purifié à partir de la souche CYL770) et 10 ou 20 mg de la protéine porteuse (exoprotéine A recombinante Δ553 i.e. rEPA) dans NaCl 100 mM et EDAC 50 mM a été préparée. Pendant le déroulement de la conjugaison qui a duré 90 min à 4°C, le pH est constamment ajusté à une valeur de 5,6 par ajout d'une solution diluée de HCl. A la fin de la réaction, le pH est neutralisé. L'antigène conjugué est ensuite dialysé contre NaCl 200 mM puis purifié par chromatographie d'exclusion de taille sur colonne sépharose Cl-4B équilibrée avec NaCl 200 mM dans un tampon phosphate 10 mM pH 7,2. Les fractions qui contenaient des conjugués (comme détecté par absorption optique à 210 nm et 280 nm) et qui sont principalement éluées avec le volume mort de la colonne, ont été rassemblées.

### Caractérisation de l'antigène conjugué

Après purification, les quantités de polysaccharide et protéine conjugués ont été estimés en tant que rapport de poids entre le polysaccharide (déterminé par quantification de O-acétyl selon la méthode de Hestrin (Hestrin S. 1949.J.Biol. Chem.180 :249-261) et la protéine (déterminé par la quantification de protéine selon la méthode de Bradford (Anal. Biochem. 1976 72,248-254)). Le niveau d'antigène non-conjugué et de protéine porteuse libre a été déterminé par électrophorèse capillaire. La taille du conjugué a été estimée par HPSEC/LS/RI/Visc.

### 5.2- Production de conjugués de type 5 (STAPH5-rEPA)

### Dépolymérisation du polysaccharide de type 5 purifié à partir de la souche CYL 1892

Avant sa conjugaison, l'antigène rPS5 purifié à partir de la souche CYL1892 est dépolymérisé selon le principe décrit dans le brevet US 6,045,805. L'antigène rPS5 est préparé à 2 mg/ml. A 20 ml de cette solution à température ambiante, sont ajoutés 55 µl d'acide ascorbique 200 mM, 5,5 µl de CuSO4 20 mM et 5,5 µl de FeS04 20 mM. La réaction est poursuivie sous agitation pendant 80 min à température ambiante. La réaction de dépolymérisation est stoppée par ajout de 2 ml d'une solution de TRIS 2 M à pH 7,0. Les réactifs de dépolymérisation sont éliminés par dialyse contre de l'eau puis le rPS5 dépolymérisé est lyophilisé. La masse moléculaire moyenne du rPS5 dépolymérisé a été estimée proche de 50 kDa par analyse HPSEC/LS/RI/Visc.

### Activation de l'antigène

40 mg d'antigène (dépolymérisé pour le polysaccharide purifié à partir de la souche CYL1892) ont été dissous dans 8 ml de NaCl 200 mM et d'acide adipique dihydrazide (ADH) 50 mM. Le pH est ajusté à 4,9 et de l'éthyldiméthylaminopropylcarbodiimide (EDAC) est ajouté à une concentration finale de 10 mM. Pendant le déroulement de l'activation qui a duré 45 min à température ambiante, le pH est constamment ajusté à une valeur de 4,9 par ajout d'une solution diluée de HCl. La réaction est stoppée par neutralisation du pH. L'antigène activé est ensuite dialysé contre NaCl 500 Mm puis contre l'eau. L'antigène activé et dialysé est alors lyophilisé. Le pourcentage de fonctionnalisation a été estimé à 1,25 et 0,9 (p/p) pour le polysaccharide purifié à partir de la souche Reynolds et pour le polysaccharide purifié et dépolymérisé à partir de la souche CYL1892, respectivement.

### Conjugaison de l'antigène

Une solution de 10 ml contenant 20 mg de l'antigène activé (et dépolymérisé pour l'antigène purifié de CYL1892) et 40 mg de la protéine porteuse (exoprotéine A recombinante, rEPA) dans NaCl 100 mM et EDAC 50 mM a été préparée. Pendant le déroulement de la conjugaison qui a duré 90 min à 4°C, le pH est constamment ajusté à une valeur de 5,6 par ajout d'une solution diluée de HCl. A la fin de la réaction, le pH est neutralisé. L'antigène conjugué est ensuite dialysé contre NaCl 200 mM puis purifié par chromatographie d'exclusion de taille sur colonne sépharose Cl-4B équilibrée avec NaCl 200 mM dans un tampon phosphate 10 mM pH 7,2. Les fractions qui contenaient des conjugués (comme détecté par absorption optique à 210 nm et 280 nm) et qui sont principalement éluées avec le volume mort de la colonne, ont été rassemblées.

### Caractérisation de l'antigène conjugué

Après purification, les quantités de polysaccharide et protéine conjugués ont été estimés en tant que rapport de poids entre le polysaccharide (déterminé par quantification de O-acétyl selon la méthode de Hestrin (Hestrin S. 1949.J.Biol. Chem.180 :249-261) et la protéine (déterminé par la quantification de protéine selon la méthode de Bradford (Anal. Biochem. 1976 72,248-254)). Le niveau d'antigène non-conjugué et de protéine porteuse libre a été déterminé par électrophorèse capillaire. La taille du conjugué a été estimée par HPSEC/LS/RI/Visc.

### Exemple 6: Evaluation de l'immunogénicité des conjugués de type 8 (PS T8-rEPA) chez la souris

Différents lots de conjugués PS T8-rEPA en utilisant du PS8 purifié à partir de la souche de type 8 Becker ont été générés par les auteurs. L'immunogénicité de ces conjugués de type 8 a pu être mise en évidence dans différentes lignées de souris, consanguines ou non consanguines, par l'analyse ELISA de la réponse humorale anti-PS8 (IgM, IgG et sous-classes d'IgG). Des études de dose-effets ont également été réalisées sur ce type de modèle animal.

Pour mettre en évidence, l'immunogénicité induite par des conjugués rPSfT8-rEPA générés en utilisant le PS8 purifié à partir de la souche recombinante CYL770, des tests animaux et des analyses immunologiques du même type que celles mises au point pour les conjugués de type 8 ayant une origine Becker (souche sauvage), ont été développés et mis en place.

### Protocole d'immunisation

**Injections :** Des souris femelles OF1 (non consanguines) (ESD - Charles River Laboratories, St Germain-sur-l'Arbresle, France) de 20-22 g sont injectées par voie sous-cutanée dans la ceinture scapulaire, à J0 (sensibilisation) puis 3 semaines après la première injection (J21) avec différentes concentrations de conjugués PS T8-rEPA générés avec du PS8 issu de la souche CYL770 cultivée en milieu M1. Les concentrations et les volumes utilisés pour chacune des injections sont décrits ci-dessous.

**Tableau 7 : Descriptif des groupes nécessaires (4 lots de conjugués) pour une étude de comparaison de l'immunogénicité induite par des conjugués de type 8 provenant de la souche Becker sauvage ou de la souche CYL770.**

| Groupes | Antigène | | Tampon de dilution | Injection | |
|---|---|---|---|---|---|
| | Désignation | Dose / souris | | Voie | Volume |
| A | rEPA (2,5 µg / souris) + sPS8 purifié de Becker (2,5 µg / souris), non conjugués | 2 x 2,5 µg | 0,9% NaCl | SC | 200 µl |
| B | rEPA (2,5 µg / souris) + rPS8f de CYL770 (2,5 µg/souris), non conjugués | 2 x 2,5 µg | 0,9% NaCl | SC | 200 µg |
| C | PS8-rEPA lot 3 (sPS8 de Becker) Prot/PS=0.16 | 2 x2,5 µg | 0,9% NaCl | SC | 200 µg |
| D | PS8-rEPA lot 4 (sPS8 de Becker) Prot/Ps = 0.51 | 2 x 2,5 µg | 0,9% NaCl | SC | 200 µl |
| E | PS8-rEPA lot 5 (rPS8f de CYL770) Prot/PS = 0.69 | 2 x 2,5 µg | 0,9% NaCl | SC | 200 µl |
| F | PS8-rEPA lot 6 (rPS8f de CYL770) Prot/PS = 1.07 | 2 x 2,5 µg | 0,9% NaCl | SC | 200 µg |
| G | rEPA (2,5 µg / souris) + rPS8 de CYL770 non fractionné (2,5 µg /souris), non conjugués | 2 x 2,5 µg | 0,9% NaCl | SC | 200 µg |

Prot/PS représente le rapport final après purification. Il indique la proportion de protéine recombinante par rapport à la proportion de polysaccharide PS8 purifié en poids/poids.

**Prélèvements de sang intermédiaires :** Environ 500 µl de sang sont prélevés au sinus retroorbitaire et collectés dans des tubes Vacutainer^{™} chez les animaux pré-immuns, avant la sensibilisation (« prime ») (J0) puis à J21, avant l'amplification (« boost ») pour chacune des souris. Pour les prélèvements intermédiaires, les souris sont anesthésiées par injection intrapéritonéale de 0,2 ml / souris d'un mélange 8 mg/ml de Ketamine et 1,6 mg/ml de Xylazine.

**Prélèvement final de sang par exsanguination :** Tous les animaux sont exsanguinés sous anesthésie générale à J35, 15 jours après l'amplification. Entre 1 ml et 1.5 ml de sang sont prélevés par animal et collectés dans des tubes Vacutainer^{™}.

Les échantillons de sang sont laissés à coaguler et à exuder pendant 3H à température ambiante (20-22°C) puis centrifugés à 4°C pendant 3 min à 6000 g puis transférer dans des tubes coniques de 1 ml Nunc et les sérums sont conservés à -20°C jusqu'à utilisation.

### Analyses des réponses anticorps

La réponse humorale anti-PS8 (titres IgG et IgM anti-PS8 à J0, J21 et J35, et titres IgG1, IgG2a et IgG3 anti-PS8 à J0 et J35 pour la dose d'immunisation déterminée au sein de l'essai) est évaluée par un test ELISA utilisant du PS8 caractérisé, purifié à partir de la souche T8 Becker comme antigène pour l'adsorption (« coating ») à une concentration de 1,5 µg/ml.

Le PS8 purifié à partir de la souche Becker, et non celui purifié à partir de la souche recombinante CYL770, a été choisi pour l'adsorption afin d'évaluer la réponse anticorps générée par les conjugués rPSfT8-rEPA (origine CYL770) contre un sPS8 purifié à partir d'une souche dite sauvage. Tous les sérums spécifiques du PS8 sont analysés par test ELISA robotisé (Zymark robot) selon la procédure suivante.

Des plaques ELISA de 96 puits (M129B Dynex) sont incubées pendant une nuit à température ambiante (20-22°C) en présence de 1 µg/ml de PS8 purifié à partir de la souche Becker dans du PBS 1X pH 7.2 (100 µl / puits). Les plaques sont lavées 4 fois avec 300 µl / puits de PBS / 0.05 % Tween 20 (PBS-Tween) à l'aide du laveur de plaques automatiques Titertek M96V Washer puis saturées pendant 1 h à 37°C avec 250 µl / puits de PBS-Tween /1% BSA. Les plaques sont lavées 4 fois selon la méthode décrite précédemment. 100-µl par puits de chacun des sérums à tester à différentes concentrations sont déposés dans la plaque. Les dilutions successives de raison 2 sont réalisées au sein même de la plaque (dilution en ligne) dans du PBS-Tween 1 % BSA. Les plaques sont incubées pendant 1 h à 37°C puis lavées 4 fois avec du PBS-Tween

100 µl / puits de conjugué anti-Ig de souris, dilué à la concentration appropriée en fonction de la classe d'Ig dans du PBS-Tween /1% BSA, sont ajoutés et les plaques sont incubées pendant 1 h à 37°C puis lavées 4 fois avec du PBS-Tween. Pour la révélation, 100 µl de la solution de TMB sont ajoutés par puits et les plaques sont incubées pendant 20 min à température ambiante (20-22°C) et à l'obscurité. La réaction enzymatique due à la peroxydase est stoppée par l'ajout de 100 µl de HCl 1 N par puits. La densité optique (DO) de chacun des puits est mesurée de 450 nm à 630 nm l'aide d'un lecteur de plaque automatique (Labsystem). Le bruit de fond (valeur moyenne sur 4 puits blancs) est soustrait aux valeurs de DO mesurées.

Les titres anti-PS8 sont calculés à l'aide du logiciel CodUnit, pour des DO dont les valeurs varient entre 0.2 et 3, par rapport à la courbe de référence donné par le sérum standard qui est présent sur chacune des plaques. Le titre Ig de ce standard, exprimé en unité ELISA arbitraire, a été préalablement déterminé sur un cumul de plusieurs mesures et correspond à la moyenne arithmétique des inverses de dilution pour une DO_{450 nm} = 1. Le seuil de détection de cet ELISA est évalué à 10 unités ELISA (1log₁₀). Les titres de chaque sérum sont exprimés en log₁₀.

### Résultats

### Titre des IgM anti-PS8

Les résultats représentés sur la Figure 2 montrent qu'une réponse significative mais non spécifique anti-PS8 est observée chez tous les animaux préimmuns (titre supérieur à 2 Log). Après la sensibilisation (J21), une augmentation marquée de la réponse spécifique IgM anti-PS8 est observée pour les 4 groupes de conjugués (~ + 2 Log) et aucune différence n'est observée entre ces 4 groupes. Pour les groupes ayant reçu du PS8 non conjugué, on observe une augmentation marquée des titres IgM anti-PS8 pour le groupe A (PS8 de Becker + rEPA non conjugué) d'environ 1 Log et pour le groupe G (PS8 natif de CYL770 + rEPA, non conjugués) d'environ 1.5 Log. Aucune augmentation des titres IgM anti-PS8 n'est observée pour le groupe B (PS8 fractionné de CYL770 + rEPA non conjugué). A J35, il n'y a pas d'effet d'amplification significative de la réponse IgM quel que soit le groupe considéré.

### Titre des IgG totales anti-PS8

Les résultats représentés sur la Figure 3 montrent que pas ou très peu de réponse IgG non spécifique anti-PS8 est observée chez les animaux préimmuns (<1.3 Log).

Après la sensibilisation (J21), une très forte réponse spécifique IgG anti-PS8 est observée pour les 4 groupes de conjugués (> + 2.3 Log), avec les moyennes de réponses comprises entre 3.5 Log et 4.1 Log. Aucune différence n'est observée entre les 4 groupes de conjugués après la sensibilisation. Cette réponse anti-PS8 est nettement supérieure à celle obtenue dans les groupes injectés avec les PS non conjugués. Cependant, on peut noter une augmentation des titres IgG anti-PS8 à J21, d'environ 1 Log, pour les groupes A (PS8 de Becker + rEPA non conjugué) et G (PS8 natif de CYL770 + rEPA non conjugué). Aucune augmentation des titres IgG anti-PS8 n'est observée pour le groupe B (PS8 fractionné de CYL770 + rEPA non conjugué).

A J35, un effet amplificateur significatif est observé uniquement pour les 4 groupes de conjugués quel que soit le groupe considéré (> + 0.6Log). Les moyennes de réponses anti-PS8 sont comprises entre 4.1 Log et 4.9Log. En considérant les moyennes de réponses, il n'y a pas de différence significative entre les conjugués issus de Becker et les conjugués issus de CYL770. Cependant, il semble que la réponse anti-PS8 obtenue avec les conjugués issus de la souche CYL770 évaluée sur PS8 purifié à partir de la souche sauvage Becker, soit plus homogène au sein d'un même groupe.

### Titres des sous-classes IgG1, IgG2a et Ig3 anti-PS8, mesurés à J35

La Figure 4 montre qu'aucune réponse non spécifique anti-PS8 n'est observée chez les animaux préimmuns (<1.2 Log).

Pour les IgG1, une très forte réponse spécifique anti-PS8 est observée pour les 4 groupes de conjugués (> 4 Log) tandis qu'aucune réponse n'est détectée pour les 3 groupes ayant reçu les différents types de polysaccharides non conjugués. Il n'y a pas de différence significative dans les moyennes des réponses antre les conjugués Becker et les conjugués CYL770, mais il semble néanmoins que la réponse obtenue avec les 2 conjugués CYL770 soit plus homogène au sein d'un même groupe.

Pour les IgG2a, la réponse est peu élevée pour les 4 groupes de conjugués (entre 2.5 Log et 2.8 Log) mais cependant significative en comparaison des réponses obtenues avec les groupes immunisés avec les PS non conjugués.

Pour les IgG3, une réponse IgG3 plus marquée est observée pour les 4 lots de conjugués en comparaison avec la réponse IgG2a. Aucune différence significative n'est observée entre les différents conjugués. Les groupes ayant reçu les PS non conjugués ne présentent pas de réponse IgG3 anti-PS8

Ces résultats montrent que les conjugués PS8-rEPA, générés à partir de PS8 purifié, issu de la souche dite sauvage Becker ou de la souche recombinante CYL770, induisent de fortes réponses anticorps spécifiques anti-PS8 dans le modèle souris OF1, comparés aux polysaccharides purifiés injectés seuls. Un effet amplificateur significatif est également observé pour ces quatre lots de conjugués. Cette réponse étant évaluée sur du PS8 purifié à partir de la souche dite sauvage Becker. Du point de vue des moyennes des réponses anticorps, aucune différence significative n'est observée entre les différents lots de conjugués, quelle que soit l'origine du PS8. Cependant, il semble que les réponses IgG totales et IgG1 anti-PS8, obtenues avec les conjugués issus de CYL770 soient plus homogènes au sein d'un même groupe.

### Exemple 7 : Evaluation de l'immunogénicité des conjugués de type 5 (PS T5-rEPA chez la souris

Différents lots de conjugués PS T5-rEPA ont été générés en utilisant du PS5 purifié à partir de la souche de type 5 Reynolds ou du rPS5 purifié à partir de la souche recombinante CYL1892 de *Staphylococcus aureus.*

L'immunogénicité de ces conjugués de type 5 a pu être mise en évidence chez la souris, par une analyse en ELISA de la réponse anticorps anti-PS5 (IgM et IgG totales). Pour comparer les réponses anticorps spécifiques induites, chacun des conjugués a été injecté à une dose optimale d'immunisation (préalablement définie dans des études d'effet-dose) de 2.5 µg / souris/ injection)..

### Protocole d'immunisation

**Injections :** Des souris femelles OF1 (non consanguines) (ESD-Charles River Laboratories, St Germain-sur-l'Arbresle, France) d'un poids de 20-22g sont injectées par voie sous cutanée dans la ceinture scapulaire à J0 (sensibilisation) puis de nouveau à J21, 3 semaines après cette première injection avec 2.5 µg /souris/ injection de conjugués PS T5-rEPA générés à partir de PS5 purifié issu de la souche Reynolds cultivée en milieu M1 ou à partir de rPS5f purifié issu de la souche CYL1892.

**Table 8: Caractéristiques des conjugués testés**

| **Désignation** | **PS5-rEPA # 7** | **PS5-rEPA # 11** | **rPS5-rEPA # 14** | **rPS5-rEPA # 15** |
|---|---|---|---|---|
| Dérivation | PS5 0273-AH III | PS5 0316-AH V | rPS5 0383F2 | rPS5 0383F2 |
| Ratio ADH/PS | 1,92% | 1,26% | 0,88% | 0,88% |
| Conditions de conjugaison | [PS5] = 2mg/ml | [PS5] = 2mg/ml | [PS5] = 2mg/ml | [PS5] = 2mg/ml |
| | Ratio Prot/PS = 0,5 | Ratio Prot/PS = 2 | Ratio Prot/PS = 2,6 | Ratio Prot/PS = 2 |
| Purification | Diafiltration CL4B | Ultrafiltration | Diafiltration CL4B | Diafiltration CL4B |
| Prot (µg/ml) | 33 | 277 | | |
| **PS(µg/ml)** | **37** | **184** | | |
| **Ratio** **Prot/PS** | **0,89** | **1,51** | **4,5** | **4.1** |
| **Mw (kDa)** | **600** | **9000** | **14 000** | **11 000** |

**Table 9: Descriptifs des groupes nécessaires à la comparaison de l'immunogénicité induite par des conjugués de type 5 dont les PS proviennent de la souche sauvage Reynolds ou de la souche recombinante CYL1892**

| Groupes | Antigène | | Tampon de dilution | Injection | |
|---|---|---|---|---|---|
| | Désignation | Dose µg (PS5) /souris | | Voie | Volume µl |
| A | rEPA 0265 (2,5 µg / souris) + PS5 0316 (2,5µg / souris), non conjugués | 2 x 2,5 | 0,9% NaCl | SC | 200 |
| B | rEPA 0265 (2,5 µg / souris) + rPS5 0383F2 (2,5µg / souris), non conjugués | 2 x 2,5 | 0,9% NaCl | SC | 200 |
| C | rPS5-rEPA **# 14** | 2 x 2,5 | 0,9% NaCl | SC | 200 |
| D | rPS5-rEPA **# 15** | 2 x 2,5 | 0,9% NaCl | SC | 200 |
| E | PS5-rEPA **# 11** | 2 x 2,5 | 0,9% NaCl | SC | 200 |
| F | PS5-rEPA **# 7** | 2 x 2,5 | 0,9% NaCl | SC | 200 |

**Prélèvements intermédiaires de sang** : Environ 200µl de sang sont prélevés au sinus rétroorbitaire et collectés dans des tubes Vacutainer^{™}. Ces prélèvements sont effectués à J0 sur les animaux préimmuns avant la sensibilisation, à J21 avant l'amplification (« boost ») pour chacune des souris individuellement. Pour les prélèvements intermédiaires, les souris sont anesthésiées par injection intrapéritonéale de 0,2 ml / souris d'un mélange de 8 mg / ml de Ketamine (Imalgene) et de 1,6 mg /ml de Xylazine (Roumpun).

**Prélèvement final de sang par exsanguination** : Tous les animaux sont exsanguinés par section carotidienne sous anesthésie générale à J35, 15 jours après le jour de l'amplification. Entre 1 ml et 1.5 ml de sang sont prélevés par animal et collectés dans des tubes Vacutainer^{™}.

Les échantillons de sang sont laissés à coaguler et à exsuder pendant 3H à 20°C-22°C, centrifugés pendant 3 min à 6000 g puis transférés dans des tubes coniques Nunc de 1 ml. Les sérums sont conservés à -20°C jusqu'à leur utilisation.

### Analyse de la réponse anticorps

La réponse humorale anti-PS5 (titres IgM et IgG anti-PS5 à J0, J21 et J35) est évaluée par un test ELISA robotisé utilisant le sPS5 purifié à partir de la souche de type 5 Reynolds comme antigène pour l'adsorption (« coating ») à une concentration de 1 µg / ml.

Le sPS5 purifié à partir de la souche Reynoldsa été choisi pour l'adsorption afin d'évaluer si les conjugués rPS5fT5-rEPA (origine CYL1892) sont capables d'induire une réponse anticorps capable de reconnaître spécifiquement du sPS5 purifié à partir d'une souche sauvage de *Staphylococcus aureus.* Tous les sérums spécifiques du PS5 sont analysés par un test ELISA robotisé (Zymark robot) selon la procédure décrite ci-dessous.

Des plaques ELISA de 96 puits (M129B Dynex) sont incubées pendant une nuit à température ambiante (20-22°C) en présence de 1 µg/ml de sPS5 purifié à partir de la souche Reynolds dans du PBS 1X pH 7.2 (100 µl / puits). Les plaques sont lavées 4 fois avec 300 µl / puits de PBS / 0,05 % Tween 20 (PBS-Tween) à l'aide du laveur automatique de plaques (Titertek M96V Washer) puis saturées pendant 1 h à 37°C avec 250 µl / puits de PBS-Tween / 1% BSA. Les plaques sont lavées 4 fois selon la méthode décrite précédemment. 100-µl par puits de chacun des sérums sont déposés dans la plaque. Les dilutions successives de raison 2 des sérums sont réalisées au sein même de la plaque (dilution en ligne) dans du PBS-Tween 1 % BSA. Les plaques sont incubées pendant 1 h à 37°C puis lavées 4 fois avec du PBS-Tween

100 µl / puits de conjugué anti-Ig de souris, dilué à la concentration appropriée en fonction de la classe d'Ig dans du PBS-Tween / 1% BSA, sont ajoutés et les plaques sont incubées pendant 1 h à 37°C puis lavées 4 fois avec du PBS-Tween. Pour la révélation, 100 µl de la solution de TMB sont ajoutés par puits et les plaques sont incubées pendant 20 min à température ambiante (20-22°C) et à l'obscurité. La réaction enzymatique due à la peroxydase est stoppée par l'ajout de 100 µl de HCl 1 N par puits. La densité optique (DO) de chacun des puits est mesurée de 450 nm à 630 nm l'aide d'un lecteur de plaque automatique (Labsystem). Le bruit de fond (valeur moyenne sur 4 puits blancs) est soustrait aux valeurs de DO mesurées.

Les titres anti-PS5 sont calculés à l'aide du logiciel CodUnit, pour des DO dont les valeurs varient entre 0,2 et 3, par rapport à la courbe de référence donné par le sérum standard qui est présent sur chacune des plaques. Le titre Ig de ce standard, exprimé en unité ELISA arbitraire, a été préalablement déterminé sur un cumul de plusieurs mesures et correspond à la moyenne arithmétique des inverses de dilution pour une DO_{450 nm} = 1. Le seuil de détection de cet ELISA est évalué à 10 unités ELISA (1log₁₀). Les titres de chaque sérum sont exprimés en log₁₀.

### RésultatsTitres des IgM anti-PS5

Les résultats représentés sur la Figure 7(a) montrent qu'une réponse significative mais non spécifique anti-PS5 est observée chez tous les animaux préimmuns (valeur moyenne des titres ~ 1.8 Log ± 0,2 Log).

Les souris ayant été injectées avec du PS5 ou du rPS5 purifié non conjugué à la rEPA, présentent une réponse IgM anti-PS5 plus faible à J21 (-0,7 Log) et à J35 (- 1,2 Log) que les souris ayant été injectées avec des conjugués.

Une réponse spécifique et significative est observée pour les 4 conjugués à J21 et à J35, avec une valeur moyenne des titres obtenue autour de 3,2 Log ± 0,2 Log et de 4 Log± 0,2 Log, respectivement.

Aucune différence significative n'a été mise en évidence entre les différents conjugués en termes de réponse IgM anti-PS5 à J21 et J35, ils présentent donc une immunogénicité similaire à celle des conjugués PS5-rEPA. De plus les IgM induites par les conjugués rPS5-rEPA sont capables de reconnaître de façon spécifique le PS5 purifié à partir d'une souche sauvage de type 5 telle que la souche Reynolds.

### Titres des IgG anti-PS5

Les résultats présentés sur la Figure 7(b) montrent qu'aucune réponse IgG anti-PS5 n'est observée chez les animaux préimmuns (valeur moyenne des titres < 1,3 Log : seuil de détection).

Une réponse IgG anti-PS5 significative est observée à J21, pour les groupes de souris ayant été injectées avec l'un ou l'autre des conjugués, avec une valeur moyenne des titres évaluée au alentour de 3,2 Log ± 0,2 Log. Un très fort effet « boost » est observé à J35 pour tous les groupes de souris ayant été injectés avec l'un ou l'autre des conjugués, avec une valeur moyenne des titres évaluée au alentour de 5 Log ± 0,2 Log.

De plus, aucune différence significative n'a été mise en évidence à J21 comme à J35 entre les 4 conjugués donc les conjugués rPS5-rEPA présentent une immunogénicité similaire à celle des conjugués PS5-rEPA. De plus les IgG induites par les conjugués rPS5-rEPA sont capables de reconnaître de façon spécifique le PS5 purifié à partir d'une souche sauvage de type 5 telle que la souche Reynolds, de la même façon que les IgG spécifiques induites par les conjugués PS5-rEPA.

Les réponses IgM et IgG anti-PS5 induites chez la souris avec les conjugués rPS5-rEPA (batch 14 et 15) sont très similaires à celles induites par les conjugués PS5-rEPA (batch 11 et 7) après le prime et après le boost. De plus, ces études ont également permis de démontrer que les conjugués rPS5-rEPA sont capables d'induire des taux élevés d'anticorps anti-PS5 reconnaissant fortement le PS5 purifié à partir d'une souche sauvage telle que la souche Reynolds de S. *aureus* et ayant une affinité pour cet antigène similaire à celle observée pour les anticorps anti-PS5 induits par les conjugués PS5-rEPA .

## Revendications

1. Composition immunogène comprenant un polysaccharide capsulaire de type 8 et/ou de type 5 selon laquelle les poids moléculaires moyens des polysaccharides capsulaires de type 8 et de type 5 sont respectivement 250 kDa et 150 kDa.

2. Composition immunogène selon la revendication 1, dans laquelle les taux de N- et O- acétylation des polysaccharides capsulaires de type 8 et de type 5 sont supérieurs à 70%.

3. Composition immunogène selon la revendication 1 ou 2, dans laquelle le polysaccharide capsulaire de type 8 est produit à partir d'une souche de S. *aureus* de type 8 dans laquelle le promoteur principal de l'opéron cap8 a été remplacé par un promoteur principal fort d'une autre souche de *S. aureus* et/ou en ce que le polysaccharide capsulaire de type 5 est produit à partir d'une souche de *S. aureus* de type 5 dans laquelle le promoteur principal de l'opéron cap5 a été remplacé par un promoteur principal fort d'une autre souche de *S. aureus.*

4. Composition immunogène selon l'une des revendications 1 à 3, dans laquelle le polysaccharide capsulaire de type 8 est produit à partir de la souche Becker dans laquelle le promoteur principal de l'opéron cap8 a été remplacé par le promoteur principal cap1 de la souche M de *S. aureus.*

5. Composition immunogène selon l'une des revendications 1 à 4, dans laquelle le polysaccharide capsulaire de type 5 est produit à partir de la souche Reynolds dans laquelle le promoteur principal de l'opéron cap5 a été remplacé par le promoteur principal cap1 de la souche M de *S. aureus.*

6. Composition immunogène selon l'une des revendications 1 à 5, dans laquelle le polysaccharide capsulaire de type 8 et/ou de type 5 a été isolé ou purifié à partir d'une souche de *S.aureus.*

7. Composition immunogène comprenant un polysaccharide capsulaire de type 8 selon l'une des revendications 1 à 4 et 6 lié à une protéine porteuse et/ou un polysaccharide capsulaire de type 5 selon l'une des revendications 1 à 3 et 5 et 6 lié à une protéine porteuse.

8. Composition immunogène selon la revendication 7, selon laquelle la molécule porteuse est l'exotoxine A de *Pseudomonas aeruginosa.*

9. Composition immunogène selon l'une des revendications 7 ou 8, dans laquelle le polysaccharide capsulaire de type 5 et/ou de type 8 a été fractionné avant d'être lié à la protéine porteuse.

10. Utilisation d'une composition immunogène selon l'une quelconque des revendications 1 à 9 pour prévenir et/ou traiter une infection à *S. aureus.*

11. Procédé de production d'un polysaccharide capsulaire de type 5 comprenant les étapes de :
a) culture d'une souche de *S. aureus* dans laquelle le promoteur principal de l'opéron cap5 a été remplacé par un promoteur principal fort d'une autre souche de *S. aureus,*
b) inactivation de la culture ainsi produite, et
c) récupération du polysaccharide capsulaire de type 5 à partir du surnageant de culture.

12. Procédé selon la revendication 11, dans lequel la souche est la souche Reynolds dans laquelle le promoteur principal de l'opéron cap5 a été remplacé par le promoteur principal cap1 de la souche M de *S. aureus.*

13. Procédé de production d'un conjugué comprenant un polysaccharide capsulaire de type 5 ou de type 8 lié à une protéine porteuse comprenant les étapes de :
a) culture d'une souche de *S. aureus* de type 5 ou de type 8 dans laquelle le promoteur principal de l'opéron cap5 ou cap8 a été remplacé par un promoteur principal fort d'une autre souche de *S. aureus,*
b) inactivation de la culture ainsi produite,
c) récupération du polysaccharide capsulaire de type 5 ou de type 8 à partir du surnageant de culture,
d) purification du polysaccharide capsulaire récupéré,
e) fractionnement du polysaccharide capsulaire purifié, et de
f) liaison à une protéine porteuse du polysaccharide capsulaire fractionné.

14. Procédé selon la revendication 13, selon lequel le polysaccharide capsulaire est un polysaccharide de type 5 et la souche de *S. aureus* est la souche Reynolds dans laquelle le promoteur principal de l'opéron cap5 a été remplacé par le promoteur principal cap1 de la souche M de *S. aureus.*

15. Procédé selon la revendication 13, selon lequel le polysaccharide capsulaire est un polysaccharide de type 8 et la souche de *S. aureus* est la souche Becker dans laquelle le promoteur principal de l'opéron cap8 a été remplacé par le promoteur principal cap1 de la souche M de *S. aureus.*

16. Procédé selon l'une des revendications 13 à 15, selon lequel la protéine porteuse est l'exotoxine A de *Pseudomonas aeruginosa.*
